# EUROPEAN PATENT SPECIFICATION

(11) **EP 2 787 958 B1**
(45) Date of publication and mention of the grant of the patent: **20.11.2019**
(21) Application number: 12815803.7
(22) Date of filing: 05.12.2012
(51) Int. Cl.: A61Q 19/08, A61Q 19/00, A61K 8/49, A61K 8/9789, A61K 36/66

(54) **CHELIDONIUM MAJUS EXTRACTS AND THEIR USE IN THE TREATMENT OF SKIN DISORDERS AND PROMOTION OF SKIN REGENERATION**
CHELIDONIUM-MAJUS-EXTRAKTE UND IHRE VERWENDUNG BEI DER BEHANDLUNG VON HAUTERKRANKUNGEN UND ZUR FÖRDERUNG DER HAUTREGENERATION
EXTRAITS DE CHELIDONIUM MAJUS ET LEUR UTILISATION DANS LE TRAITEMENT DE TROUBLES CUTANÉS ET LA PROMOTION DE LA RÉGÉNÉRATION CUTANÉE

(30) Priority: 05.12.2011 IT MI20112215
(43) Date of publication of application: 15.10.2014
(73) Proprietor: PURYTRA FARMACEUTICI S.P.A., 20135 Milano (IT)
(72) Inventor: PIETRA, Daniele, I-56017 San Giuliano Terme (IT); BORGHINI, Alice, I-56017 San Giuliano Terme (IT); DEL CORSO, Simone, I-56122 Pisa (IT); IMBRIANI, Marcello, I-27100 Pavia (IT); BIANUCCI, Anna, I-56122 Pisa (IT)
(74) Representative: Zaccaro, Elisabetta
(86) International application number: PCT/IB2012/056987
(87) International publication number: WO 2013/084162

(56) References cited:
- CH-A5- 671 336
- DE-A1- 4 031 960
- DE-A1- 19 849 107
- US-A- 5 997 876
- DATABASE WPI Week 200660 Thomson Scientific, London, GB; AN 2006-583786 XP002681569, -& KR 2005 0080882 A (CHAI K Y) 18 August 2005 (2005-08-18) & KIPO: "KR1020050080882 Patent File Wrapper Information", Korean Intellectual Property Office , 8 August 2005 (2005-08-08), Korea Retrieved from the Internet: URL:http://kposd.kipo.go.kr:8088/kpion/UP_ A01_PatentSearchFileWrapperPdfFrame.jsp?LN =EN&filename=temp/out_PDF_cw_gaz_p_1020040 009047_EN.pdf&AN=1020040009047&DOCTYPE=2&R SNO=null [retrieved on 2012-08-06]
- DATABASE WPI Week 200741 Thomson Scientific, London, GB; AN 2007-430033 XP002681570, & KR 2006 0122488 A (KOREAN COSMETICS CO LTD) 30 November 2006 (2006-11-30)
- ANONYMOUS: "Herbasol TM Extract Celandine", 20020123, no. Article No. 219350/12, 23 January 2002 (2002-01-23), pages 1-6, XP007920910,
- Á SÁRKÖZI ET AL: "Alkaloid Composition of Chelidonium majus L. Studied by Different Chromatographic Techniques", CHROMATOGRAPHIA ; AN INTERNATIONAL JOURNAL FOR RAPID COMMUNICATION IN CHROMATOGRAPHY, ELECTROPHORESIS AND ASSOCIATED TECHNIQUES, VIEWEG VERLAG, WI, vol. 63, no. 13, 8 March 2006 (2006-03-08) , pages S81-S86, XP019388533, ISSN: 1612-1112, DOI: 10.1365/S10337-006-0728-7
- FULDE G, AND WICHTL M: "Analytik von Schöllkraut - Das Hauptalkaloid ist Coptisin", DEUTSCHE APOTHEKER ZEITUNG, vol. 134, no. 12, 24 March 1994 (1994-03-24), pages 1031-1035, XP001526456,
- FRANCA TOMÈ ET AL: "Distribution of alkaloids in Chelidonium majus and factors affecting their accumulation", PHYTOCHEMISTRY, vol. 40, no. 1, 1 September 1995 (1995-09-01), pages 37-39, XP055034767, ISSN: 0031-9422, DOI: 10.1016/0031-9422(95)00055-C
- YUE GU ET AL: "Simultaneous determination of seven main alkaloids of Chelidonium majus L. by ultra-performance LC with photodiode-array detection", JOURNAL OF SEPARATION SCIENCE, 22 February 2010 (2010-02-22), pages NA-NA, XP055034710, ISSN: 1615-9306, DOI: 10.1002/jssc.200900690
- TÁBORSKÁ EVA ET AL: "Separation of Alkaloids in Chelidonium majus L. by Reversed Phase HPLC", PLANTA MEDICA, THIEME VERLAG, DE, vol. 60, no. 4, 1 January 1994 (1994-01-01), pages 380-381, XP009161785, ISSN: 0032-0943
- BANDELIN F.J. AND MALESH W.: "Alkaloids of Chelidonium majus, L., Leaves and Stems I: dl-Tetrahydrocoptisine", JOURNAL OF THE AMERICAN PHARMACEUTICAL ASSOCIATION, vol. XLV, no. 10, 1 October 1956 (1956-10-01), pages 702-704, XP009161784, ISSN: 0003-0465
- HÉLÈNE GUINAUDEAU ET AL: "The Protopine Alkaloids", JOURNAL OF NATURAL PRODUCTS, vol. 45, no. 3, 1 May 1982 (1982-05-01), pages 237-246, XP055085743, ISSN: 0163-3864, DOI: 10.1021/np50021a001
- DATABASE CAPLUS [Online] CHEMICAL ABSTRACTS SERVICE, COLUMBUS, OHIO, US; 14 October 2002 (2002-10-14), Di Donna, Giuseppe: "Topical composition for the removal of tattoos", XP002715595, Database accession no. 2002:779944

## Description

### Field of the invention

The present invention concerns particular extracts of *Chelidonium majus* and a novel process for obtaining them.

The invention further concerns cosmetic and pharmaceutical compositions and medical devices containing said extracts and the use of extracts in the medical and cosmetic fields for the treatment of skin disorders and for skin regeneration.

Furthermore, the invention concerns a novel use of isoquinoline alkaloids and compositions containing them.

### State of the art

Human skin consists principally of three layers: an upper thinner layer, the epidermis, a thicker intermedial layer, the dermis, and a lower layer of subcutaneous tissue.

Keratinocytes are the most abundant cell types in epidermis (75-80% of the total number of human epidermal cells), while the dermis mainly consists of fibroblasts.

Furthermore, the skin is extensively vascularised with a dense network of arterial and venous vessels consisting of vascular endothelial cells.

The skin has various regeneration and repair mechanisms that intervene both under physiological conditions and in the presence of lesions. During the tissue repair process following a skin lesion, numerous cellular and biochemical processes intervene that are capable of restoring the physiological condition of the injured tissue. These processes include the modulation of proliferation, migration, viability and cell metabolism in general; they affect mitochondrial dehydrogenase activities, the concentration of free radicals and the regulation of the cellular and extracellular oxidation and reduction environment, the modulation of the concentrations of intracellular and intercellular mediators, such as cytokines, and the modulation of collagen production.

It is known that changes in the aforementioned processes are involved in numerous skin regeneration and repair dysfunctions, such as skin ageing, skin inflammation, skin irritation, the formation of wrinkles, other than in the reactions to wounds, sores, burns and skin lesions in general, and in the occurrence of fibrosis and fibrotic conditions, scars, hypertrophic scars, atrophic scars, acne scars, scarring effects in general, stretch marks, acne, dermatous lesions and eczema. [Hernandez - Martinez, Repair, regeneration and fibrosis, in Pathology, ed. E. Rubin, J.L. Farber, Philadelphia, Lippincott, 1999; Satish L and Kathju S, Dermatology Research and Practice, 2010, Article ID 790234, 11 pages].

With the aim of promoting regeneration and repair processes in lesions associated with pathologies and other conditions concerning the skin, it is desirable to stimulate cell viability (revitalizing effect), reduce the concentration of free radicals (antiradical and skin protective effect), promote the wound healing process, by stimulating the proliferation and migration of fibroblasts, keratinocytes and skin vascular endothelial cells. In addition it is desirable to reduce the concentration of interleukin 1 beta, antagonize the inflammatory process and oxidative damage and antagonize the fibrotic process.

*Chelidonium majus* (*C. majus*) is a perennial herbaceous plant that grows spontaneously in woodlands around the Mediterranean. It belongs to the genus *Chelidonium* and to the Papaveraceae family. It reaches a height of 30-90 cm (Bruni A. (1999), Farmacognosia generate e applicata, PICCINNUOVA LIBRARIA SpA Padova, pages 300-301.)

*Chelidonium majus* contains numerous bioactive substances, including compounds belonging to the following chemical classes: alkaloids, acids, lactones, carotenoids, vitamins, flavonoids, phytosteroids, saponins and proteins, many of which have been isolated and analyzed analytically using chromatographic methods. [Kim HK et al., Journal of Pharmaceutical Sciences. 1969, 58, 372-374; Slavik J and Slavikova L, Collection of Czechoslovak Chemical Communications. 1977, 42, 2686-2693; Slavik J et al. Collection of Czechoslovak Chemical Communications. 1965, 30, 3697-3704; Tin-Wa M et al., Journal of Natural Products. 1972, 35, 87-89 (1972)]. The isoquinoline alkaloids are particularly abundant in *C. majus.*

Isoquinoline alkaloids are natural basic compounds characterized by the presence of an isoquinoline-like scaffold. In some cases the isoquinoline nucleus is reduced (hydrogenated), and/or part of the heterocycle is open. The isoquinoline alkaloids include the compounds listed below: berberine, dihydroberberine, tetrahydroberberine, canadine, protopine, allocryptopine, dihydrocoptisine,
coptisine, stylopine, chelidonine, sanguinarine and chelerythrine.

*C. majus* is rich in the following isoquinoline alkaloids: protopine, coptisine, stylopine, berberine, chelidonine, sanguinarine and chelerythrine [Gu et al. Journal of Separation Science. 2010, 33, 1004-1009].

DE19849107 relates to a method for removing undesired toxic benzyl, isoquinoline, phenanthrene, berberine and benzophenanthridine alkaloids from plant preparations, wherein the Chelidonium majus plant (irrespective of the part of the plant) is extracted for 3 hours with 70% w / w ethanol in the ratio 1:10 at a temperature of 40° C to 50° C such as alkaloids.

Known extracts of *Chelidonium majus* have the disadvantage of having a high number of chemical components of natural origin, that can irritate the skin and cause a hypersensitivity reaction, affecting the immune system and resulting in tissue changes that can cause serious pathologies.

The scope of the present invention is therefore to provide particular extracts of *Chelidonium majus,* that are clinically safe and can be used to treat various skin conditions, such as skin ageing, skin inflammation, skin irritation, wrinkles, wounds, sores, burns and skin lesions in general, fibrosis and fibrotic conditions, scars, hypertrophic scars, atrophic scars, acne scars, the effects of scarring in general, stretch marks, acne, dermatous lesions and eczema, that do not have the aforementioned drawbacks of the known compositions for the same application.

### Summary of the invention

The scope indicated above has been achieved by means of an extract of *Chelidonium majus* obtainable by the process according to claim 1.

Indeed, it has been found that the isoquinoline alkaloids and/or the extracts of the invention, at a given dose and for a given period of time, demonstrate one or more of the following effects:
- free radical scavenger activity,
- modulation of mitochondrial dehydrogenase activity,
- modulation of cell proliferation,
- modulation of collagen production,
- changes in cell morphology,
- modulation of IL-1β production,
- modulation of IL-6 production.

From said effects of the substances of the invention, it is possible to infer uses in the fields of medicine and cosmetics and/or toxic effects, as described below.

### Use in the fields of medicine and cosmetics

Antiradical (skin protective): this is deduced from the free radical scavenger effects.

Revitalizing: this is deduced from the increased mitochondrial dehydrogenase effect.

Antifibrotic (general anti-scarring, protection against atrophic scarring, hypertrophic scarring, acne scarring): this is deduced from the effect of antagonizing the stimulation of collagen induced by 34 ng/ml TGF-β1 and the effect of antagonizing the stimulation of IL-1β and/or IL-6 induced by 34 ng/ml TGF-β1 and from the inhibition of production of IL-1β and/or IL-6 under standard growth conditions.

Cytoprotective, with regard to oxidative damage (antioxidant, anti-inflammatory and anti-ageing): this is deduced from antagonising the stimulation of IL-1β and/or IL-6 induced by 100 µM H₂O₂ and from the absence of changes in cell morphology similar to the ones due to cell senescence.

Wound healing: this is deduced from the effect of inhibiting the production of IL-1β and by the effect of stimulating cell proliferation under standard growth conditions.

### Toxic effects

Cytotoxicity: this is deduced from the effect of reducing mitochondrial dehydrogenase activity or from the presence of morphologies characteristic of cell senescence.

Irritation: this is deduced from the effect of increased production of IL-6 and/or IL-1β under standard growth conditions.

Therefore, for each isoquinoline alkaloid and/or *C. majus* extract analyzed, the following properties have been surprisingly discovered:

### Berberine

### Mitochondrial dehydrogenase

Mitochondrial dehydrogenase activity is increased with respect to the control at concentrations of 1 and 10 µM, and this increase is more marked when the exposure time is 42 h compared to 18 h.

### Collagen

In the presence of 100 µM H₂O₂, it antagonizes the inhibition of collagen production induced by H₂O₂, at concentrations of both 1 µM and 10 µM. On the contrary, it antagonizes the stimulation of collagen induced by 34 ng/ml TGF-β1 at concentrations of both 1 µM and 10 µM.

### IL-6

Under standard conditions, at concentrations of 1-10 µM in the presence of 100 µM H₂O₂ and in the presence of 34 ng/ml TGF-β1, it demonstrates a tendency to reduce the production of IL-6 in a dose-dependent manner.

### Dihydroberberine

### Anti-radical

It shows anti-radical effects at a concentration of 1 mM.

### Mitochondrial dehydrogenase

It shows no significant effects on mitochondrial dehydrogenase activity at concentrations below 0.1 µM. Mitochondrial dehydrogenase activity is increased with respect to the control at concentrations of 1 and 10 µM, and this increase is more marked when the exposure time is 42 h compared to 18 h. Mitochondrial dehydrogenase activity is reduced at higher concentrations (100 µM).

### Collagen

Under standard conditions, at concentrations of 1-10 µM and in the presence of 100 µM H₂O₂, it stimulates collagen production in a dose-dependent manner. On the contrary, it antagonizes the stimulation of collagen induced by 34 ng/ml TGF-β1, and said antagonistic action is more marked at a concentration of 1 µM than at 10 µM. IL-1β

Under standard conditions, at concentrations of 1-10 µM and in the presence of 100 µM H₂O₂, it demonstrates a tendency to increase IL-1β production in a dose-dependent manner. However, it antagonizes the stimulation of IL-1β induced by 34 ng/ml TGF-β1. Said antagonistic action is greater at a concentration of 1 µM compared to 10 µM.

### IL-6

Under standard conditions, at concentrations of 1-10 µM and in the presence of 100 µM H₂O₂, it demonstrates significant effects in modulating IL-6 production. Furthermore, is demonstrates a tendency to antagonise the stimulation of IL-6 induced by TGF-β1. Said antagonistic action is greater at a concentration of 1 µM compared to 10 µM.

### Coptisine

### Collagen

Under standard conditions, at concentrations of 1-10 µM in the presence of 100 µM H₂O₂ and in the presence of 34 ng/ml TGF-β1, it stimulates collagen production in a dose-dependent manner.

### Mitochondrial dehydrogenase

It shows no significant effects on mitochondrial dehydrogenase activity at concentrations below 1 µM. IL-1β

Under standard conditions, at concentrations of 1-10 µM in the presence of 100 µM H₂O₂ and in the presence of 34 ng/ml TGF-β1, it demonstrates a tendency to increase IL-1β production in a dose-dependent manner.

### IL-6

Under standard conditions, at concentrations of 1-10 µM in the presence of 100 µM H₂O₂ and in the presence of 34 ng/ml TGF-β1, it demonstrates a tendency to increase IL-6 production in a dose-dependent manner.

### Stylopine

### Mitochondrial dehydrogenase

It shows no significant effects on mitochondrial dehydrogenase activity at concentrations below 1 µM. Mitochondrial dehydrogenase activity is increased with respect to the control at a concentration of 10 µM when the exposure time is 42 h. At higher concentrations (100 µM), a less marked stimulation of mitochondrial dehydrogenase activity is observed with respect to 10 µM. When the exposure time is 18 h, a non-significant tendency to stimulate mitochondrial dehydrogenase activity in a dose-dependent manner is observed at concentrations between 1 and 100 µM.

### Proliferation

At a concentration of 1 µM, it demonstrates a tendency to stimulate cell proliferation.

### Collagen

In the presence of 100 µM H₂O₂, it has a tendency to inhibit collagen production, at concentrations of both 1 µM and 10 µM. Furthermore, it antagonizes the stimulation of collagen induced by 34 ng/ml TGF-β1 at concentrations of both 1 µM and 10 µM. IL-1β

Under standard conditions, at concentrations of 1-10 µM, it inhibits the production of IL-1β, and also antagonizes the production of IL-1β induced by 100 µM H₂O₂ or by 34 ng/ml TGF-β1.

### IL-6

Under standard conditions, at concentrations of 1-10 µM, it inhibits the production of IL-6, and also antagonizes the production of IL-6 induced by 100 µM H₂O₂ or by 34 ng/ml TGF-β1.

### Canadine

### Mitochondrial dehydrogenase

It shows no significant effects on mitochondrial dehydrogenase activity at concentrations below 0.1 µM. On the other hand, activity has a tendency to increase in a dose-dependent manner with respect to the control at concentrations between 0.1 and 100 µM, when the exposure time is both 18 and 42 h.

### Collagen

Under standard conditions, at a concentration of 1 µM it shows no evident effect on the modulation of collagen production, while at a concentration of 10 µM it demonstrates a collagen production stimulation effect. On the other hand, it inhibits collagen production in the presence of 100 µM H₂O₂. Said inhibition is greater at a concentration of 1 µM compared to 10 µM. Furthermore, it antagonizes the stimulation of collagen induced by 34 ng/ml TGF-β1 at concentrations of both 1 µM and 10 µM. Said inhibition is greater at a concentration of 1 µM compared to 10 µM. IL-1β

Under standard conditions, at a concentration of 1 µM, it inhibits the production of IL-1β, and also antagonizes the production of IL-1β induced by 100 µM H₂O₂ or by 34 ng/ml TGF-β1. Under standard conditions, at a concentration of 10 µM, it stimulates the production of IL-1β, while it antagonizes the production of IL-1β induced by 100 µM H₂O₂ or by 34 ng/ml TGF-β1, even if to a lesser extent than at a concentration of 1 µM.

### IL-6

Under standard conditions, at concentrations of 1-10 µM, it inhibits the production of IL-6. It also antagonizes the production of IL-6 induced by 100 µM H₂O₂ or by 34 ng/ml TGF-β1. These inhibitory or antagonistic effects are higher at a concentration of 1 µM compared to a concentration of 10 µM.

### Chelerythrine

### Mitochondrial dehydrogenase

At concentrations higher than 1 nM, the predominant effect shown is an inhibition of mitochondrial dehydrogenase activity. This doesn't happen when it is administered at a concentration of 1 µM for 18 h. Under this condition a stimulation of mitochondrial dehydrogenase activity is observed.

### Proliferation

At a concentration of 1 µM it demonstrates a tendency to stimulate cell proliferation, while at a concentration of 10 µM it causes a marked inhibition of proliferation.

### Collagen

Under standard growth conditions, at a concentration of 1 µM it demonstrates a tendency to inhibit collagen production. In addition, at a concentration of 1 µM, it antagonizes the stimulation of collagen induced by 34 ng/ml TGF-β1. On the other hand, under standard conditions, at a concentration of 10 µM in the presence of 100 µM H₂O₂ and in the presence of 34 ng/ml TGF-β1, it stimulates collagen production. This tendency to stimulate collagen production is also observed at a concentration of 1 µM in the presence of 100 µM H₂O₂. IL-1β

Under standard conditions, at a concentration of 1 µM, it inhibits the production of IL-1β, and also antagonizes the production of IL-1β induced by 100 µM H₂O₂ or by 34 ng/ml TGF-β1. On the other hand, at a concentration of 10 µM, both under standard conditions and in the presence of 100 µM H₂O₂ and 34 ng/ml TGF-β1, it stimulates IL-1β production.

### IL-6

Under standard conditions, at a concentration of 1 µM, it inhibits the production of IL-6, and also antagonizes the production of IL-6 induced by 100 µM H₂O₂ or by 34 ng/ml TGF-β1. On the other hand, at a concentration of 10 µM, both under standard conditions and in the presence of 100 µM H₂O₂ and 34 ng/ml TGF-β1, it stimulates IL-6 production.

### Sanguinarine

### Mitochondrial dehydrogenase

At concentrations higher than 1 µM it causes a marked inhibition of mitochondrial dehydrogenase activity when administered both for 18 h and for 42 h.

### Proliferation

At a concentration of 1 µM it demonstrates dose-dependent inhibition of cell proliferation while at a concentration of 10 µM it causes complete cell death.

### Collagen

Under standard conditions, at concentrations of 1-10 µM in the presence of 100 µM H₂O₂ and in the presence of 34 ng/ml TGF-β1, it stimulates collagen production in a dose-dependent manner.

### Cell morphology

At a concentration of 1 µM the few cells present take on a senescent morphology. They have a similar appearance in the presence of 100 µM H₂O₂ and 34 ng/ml TGF-β1. At a concentration of 10 µM there is a complete absence of cells. IL-1β

At concentrations of 1-10 µM, both under standard conditions and in the presence of 100 µM H₂O₂ and 34 ng/ml TGF-β1, it stimulates IL-1β production in a dose-dependent manner.

### IL-6

At concentrations of 1-10 µM, both under standard conditions and in the presence of 100 µM H₂O₂ and 34 ng/ml TGF-β1, it stimulates IL-6 production in a dose-dependent manner.

### Chelidonine

### Mitochondrial dehydrogenase

At concentrations of less than 0.1 µM it demonstrates no significant effects on mitochondrial dehydrogenase activity, and this absence of effects is also observed at a concentration of 1 µM when the exposure time is 18 h. However, with increased exposure time a significant inhibition of mitochondrial dehydrogenase activity is observed, at the same concentration of 1 µM. In general, the inhibitory tendency increases with increasing concentration and exposure time.

### Proliferation

At a concentration of 1 µM it demonstrates no evident ability to modulate cell proliferation, while at a concentration of 10 µM it causes a marked inhibition of proliferation.

### Collagen

Under standard growth conditions, at a concentration of 1 µM it demonstrates no evident ability to modulate collagen production. However, again at a concentration of 1 µM, in the presence of 100 µM H₂O₂ it has a tendency to inhibit collagen production. Furthermore, in the presence of 34 ng/ml TGF-β1 it has a tendency to antagonize collagen stimulation induced by TGF-β1. On the other hand, under standard conditions, at a concentration of 10 µM in the presence of 100 µM H₂O₂ and 34 ng/ml TGF-β1, it stimulates collagen production.

### Cell morphology

No changes in cell morphology are observed with respect to the control at a concentration of 1 µM. Very obvious changes in cell morphology are observed at a concentration of 10 µM, with the appearance of a senescence-related morphology.

### IL-1β

Under standard conditions, at a concentration of 1 µM, it inhibits the production of IL-1β, and also antagonizes the production of IL-1β induced by 100 µM H₂O₂ or by 34 ng/ml TGF-β1. On the other hand, at a concentration of 10 µM, both under standard conditions and in the presence of 100 µM H₂O₂ and 34 ng/ml TGF-β1, it stimulates IL-1β production.

### IL-6

Under standard conditions, at a concentration of 1 µM, it inhibits the production of IL-6, and also antagonizes the production of IL-6 induced by 100 µM H₂O₂ or by 34 ng/ml TGF-β1. On the other hand, at a concentration of 10 µM, both under standard conditions and in the presence of 100 µM H₂O₂ and 34 ng/ml TGF-β1, it stimulates IL-6 production.

### Protopine

### Mitochondrial dehydrogenase

It shows no significant effects on mitochondrial dehydrogenase activity at concentrations below 100 µM.

### Proliferation

At a concentration of 10 µM, it demonstrates a tendency to stimulate cell proliferation.

### Collagen

At a concentration of 10 µM, under standard conditions and in the presence of 100 µM H₂O₂, it has a tendency to inhibit collagen production. Furthermore, it has a tendency to antagonise collagen stimulation induced by 34 ng/ml TGF-β1.

### IL-1β

Under standard conditions, at a concentration of 10 µM, it inhibits the production of IL-1β, and also antagonizes the production of IL-1β induced by 100 µM H₂O₂ or by 34 ng/ml TGF-β1.

### IL-6

Under standard conditions, at a concentration of 10 µM, it inhibits the production of IL-6, and also antagonizes the production of IL-6 induced by 100 µM H₂O₂ or by 34 ng/ml TGF-β1.

### Pa_f_Gli_20°C_28d and Fi_f_Gli_20°C_21d

At a concentration of 0.1%, the extracts have the following alkaloid composition (please refer to the HPLC analysis):

| | |
|---|---|
| Coptisine | 1 µM |
| Stylopine | 1 µM |
| Berberine | 0.1 µM |
| Protopine | 0.01 µM |
| Chelidonine | 0.01 µM in Pa_f_Gli_20°C_28d, 0.0001 µM in Fi_f_Gli_20°C_21d |
| Sanguinarine | 0.00001 µM |
| Chelerythrine | 0.00001 µM |

The alkaloids mostly present in the extracts are therefore stylopine and coptisine, while the other alkaloids are present in concentrations less than one tenth (berberine) and less than one hundredth of the concentration of stylopine and coptisine. Consequently, it may be speculated that the main effects of the extracts are derived from a combination of the effects of stylopine and coptisine, included the anti-radical effects that are characteristics of the extracts (that may be observed from the anti-radical activity assay results).

### Anti-radical

They demonstrate anti-radical effects.

### Mitochondrial dehydrogenase

The extracts show no significant effects on mitochondrial dehydrogenase activity at concentrations below 0.001%. However, activity increases with respect to the control at a concentration of 0.1%. At a concentration of 1%, a less marked stimulation of mitochondrial dehydrogenase activity is observed with respect to a concentration of 0.1%.

### Proliferation

At a concentration of 0.1%, the extracts demonstrate no obvious effect on modulating cell proliferation.

### Collagen

At a concentration of 0.1%, the extracts demonstrate no obvious effect on modulating collagen production under standard conditions. However, they antagonize the inhibition of collagen induced by 100 µM H₂O₂. On the other hand, they antagonize the stimulation of collagen induced by 34 ng/ml TGF-β1. IL-1β

At a concentration of 0.1% under standard conditions, the extracts demonstrate no obvious effect on modulating IL-1β production. However, they antagonize the IL-1β production induced by 100 µM H₂O₂ or by 34 ng/ml TGF-β1.

### IL-6

At a concentration of 0.1% under standard conditions, the extracts demonstrate no obvious effect on modulating IL-6 production. However, they antagonize the IL-6 production induced by 100 µM H₂O₂ or by 34 ng/ml TGF-β1.

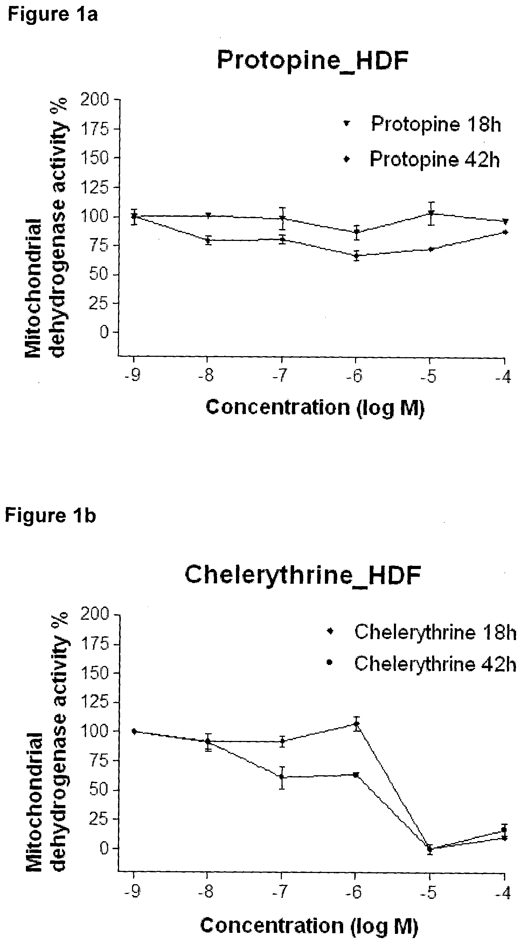

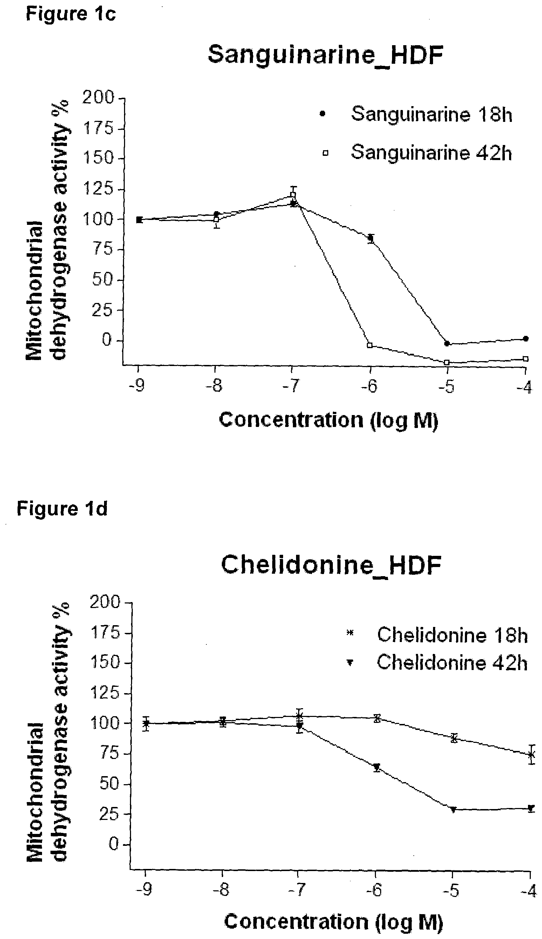

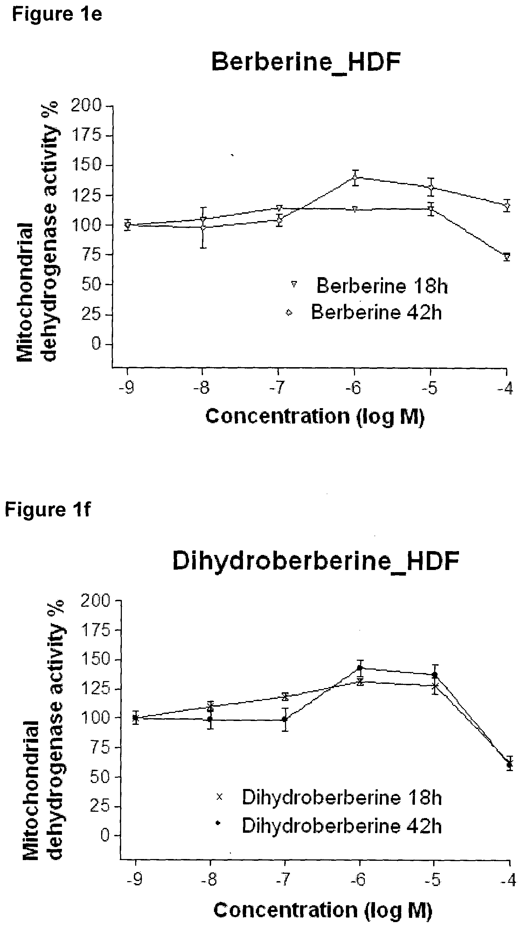

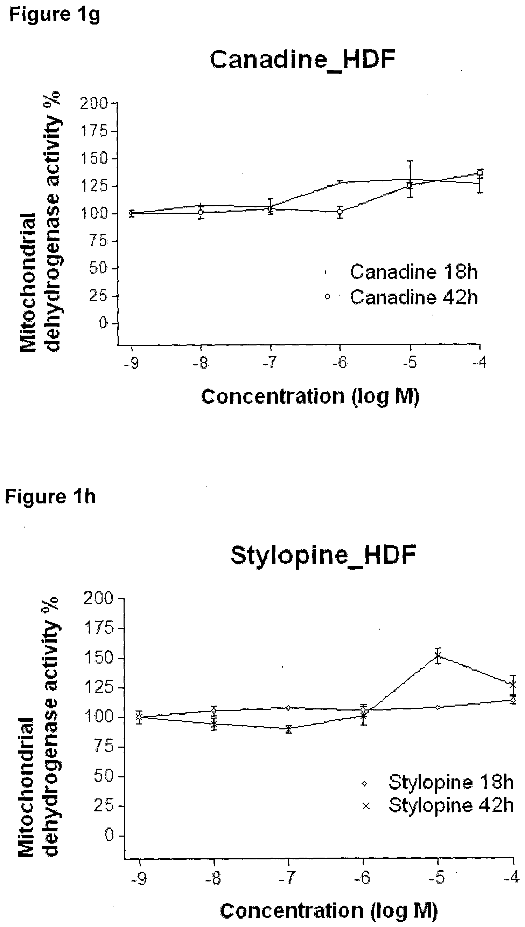

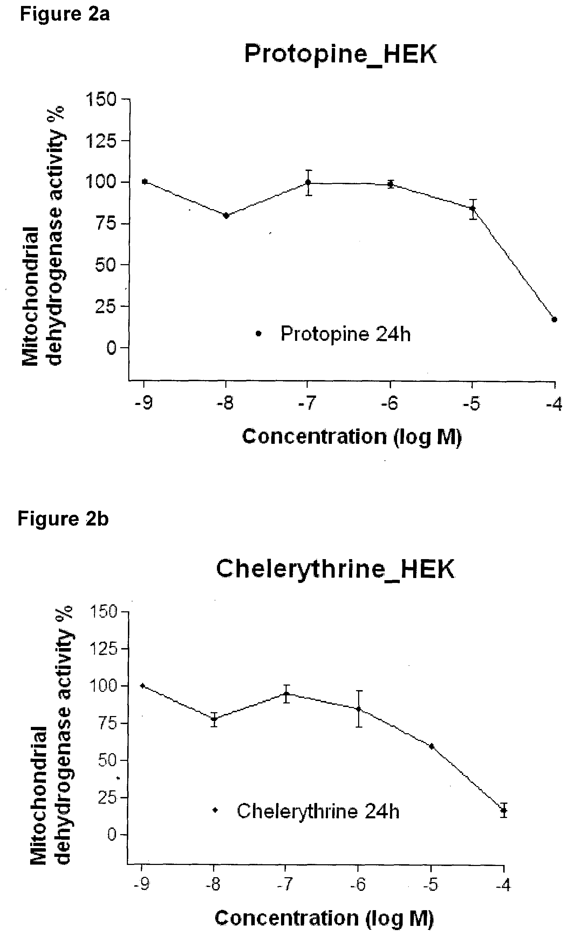

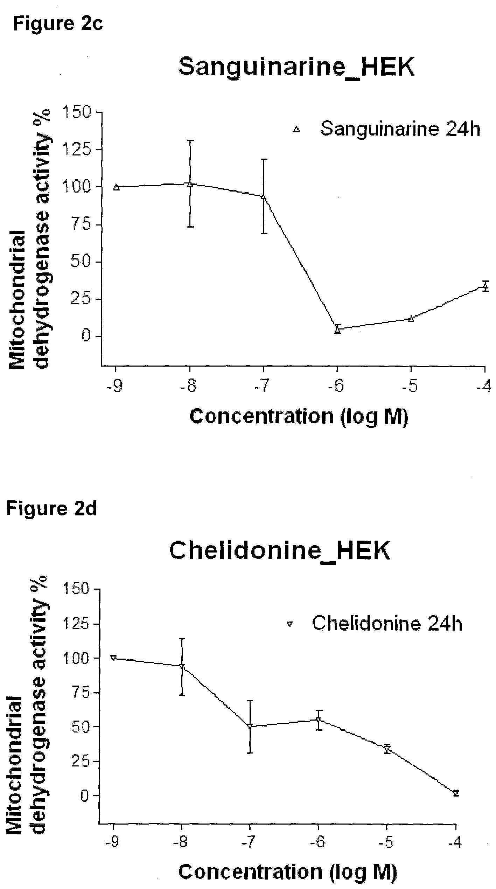

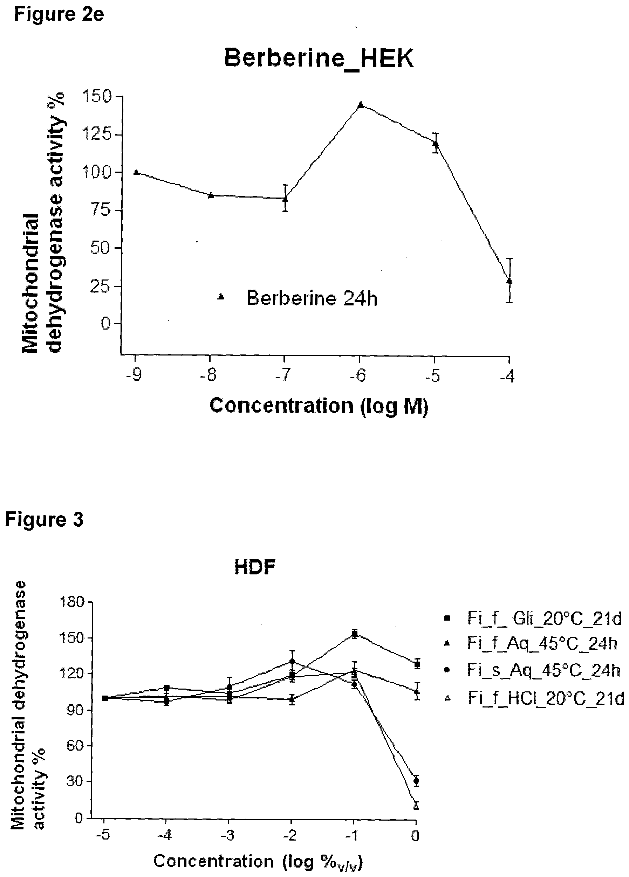

Therefore, the results of this research have lead the authors to the following conclusions and to the identification of uses in the fields of medicine and cosmetics for the substances of the invention
- Among the chemical compounds from C. majus, dihydroberberine has demonstrated the highest anti-radical activity, with an IC₅₀ value of 62 µM.
- The best solvents for achieving anti-radical action are propylene glycol and aqueous acid solution. Furthermore, fresh flowers are particularly preferred.
- The fresh flower glycol extract ***Fi_f_Gli_20°C_21d*** is particularly interesting, since at a concentration of approx. 1% it demonstrates very high antioxidant and anti-radical activity. Furthermore, at this concentration, this extract exerts no cytotoxic activity on fibroblasts. On the other hand, at concentrations of 0.01% to 1%, it demonstrates stimulation of mitochondrial dehydrogenase activity. This result makes it a useful ingredient for cosmetic formulations with antioxidant and anti-radical regenerating characteristics (anti-ageing). The optimal concentration for fresh flower glycol extract will therefore be 1%.
- The anti-radical activity of the extracts is not directly related to the presence of one or more particular *C. majus* alkaloids. Indeed, the alkaloid with a major anti-radical activity, dihydroberberine, is present in only trace amounts in the extracts.
- The pharmacological or toxic effects of the substances depend on the dosage and the duration of exposure to the dose of the substance (e.g. 1 and 10 µM chelerythrine).
- The isoquinoline alkaloids chelerythrine, sanguinarine and chelidonine demonstrate marked inhibitory power toward the mitochondrial dehydrogenase activity of skin fibroblast cells (see Figs. 1b, 1c, 1d respectively) and/or keratinocytes (see Figs. 2b, 2c, 2d respectively).
- It has also been surprisingly found that the isoquinoline alkaloids dihydroberberine, canadine and stylopine have the ability to stimulate mitochondrial dehydrogenase activity in fibroblast cells at low dosage, and on the other hand, have the ability to inhibit mitochondrial dehydrogenase activity at higher doses (see Figs. 1f, 1g, 1h and Fig. 1h respectively). Furthermore, berberine has demonstrated the ability to stimulate mitochondrial dehydrogenase activity in fibroblasts, keratinocytes and vascular endothelial cells at low doses, and on the other hand, has demonstrated the ability to inhibit mitochondrial dehydrogenase activity at higher doses (Figs. 1e and 2e).

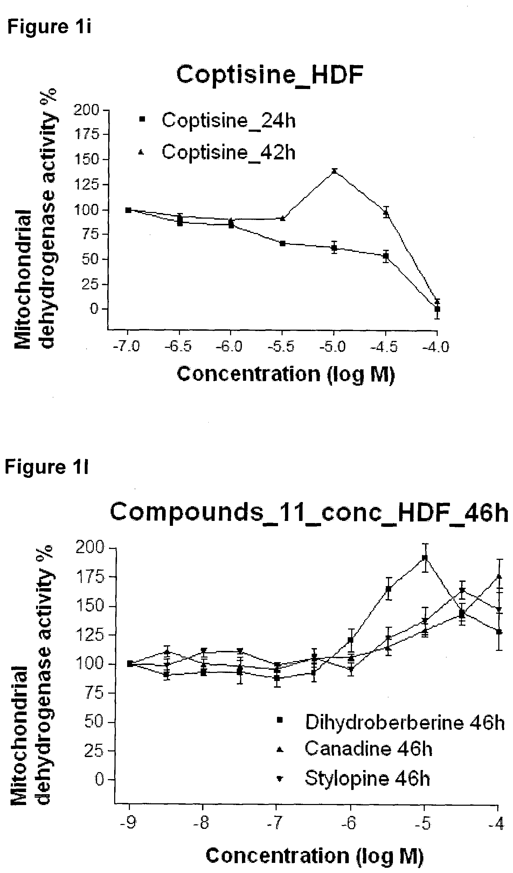

Furthermore, it has been found that the isoquinoline alkaloid coptisine demonstrates mostly inhibitory effects on mitochondrial dehydrogenase activity in fibroblasts, and to a lesser extent, stimulatory effects on mitochondrial dehydrogenase activity in said cells when the compound is administered at low dose for a prolonged period of time (Fig. 1i).

Thus, combining the surprising findings mentioned above with what is known from the state of the art, it can be predicted that the use of an extract or medicinal product or cosmetic or medical device containing stylopine, canadine, dihydroberberine, dihydrocoptisine and berberine at low doses will produce a stimulatory effect on mitochondrial dehydrogenase activity in fibroblast cells.

It may be deduced that said modulation of mitochondrial dehydrogenase activity will have a beneficial effect in the treatment of skin regeneration and repair dysfunctions.
- Reduced mitochondrial dehydrogenase activity is associated with reduced cell proliferation, and *vice versa* (see for example the effects produced by 10 µM sanguinarine, chelidonine, chelerythrine and coptisine)
- Increased cell proliferation can also occur in the absence of a significant increase in mitochondrial dehydrogenase activity (see for example the effects produced by 10 µM protopine)
- Reduced cell proliferation, and thus reduced mitochondrial dehydrogenase activity, is associated with increased cell collagen production
- The modulation of collagen production can also occur with those substances that do not demonstrate obvious effects on the modulation of cell proliferation or mitochondrial dehydrogenase activity
- The increased cell proliferation is associated with antagonism of the cell collagen production stimulated by TGF-β1
- Modulation of cell IL-1β production is associated with cell collagen production
- Reduced cell proliferation, and thus mitochondrial dehydrogenase activity, is associated with increased cell IL-6 production
- Reduced IL-6 production, or antagonism of the stimulation of IL-6 production induced by TGF-β1 or H₂O₂, can also occur with those substances that demonstrate no obvious effects on the modulation of cell proliferation or mitochondrial dehydrogenase activity
- When discussing the production of collagen, IL-6 and IL-1β, it is important to refer to cell proliferation as it can be evaluated by the number of cells actually present in the assay well, and not to refer to cell viability as assessed by mitochondrial dehydrogenase activity. Indeed, there are substances, such as sanguinarine for example, that strongly inhibit cell proliferation, and, thus, also the **absolute** levels of collagen and interleukins are notably reduced in the assay. If however, collagen and interleukin levels are related to the effective number (low) of cells present, the collagen and interleukins produced *per cell* increase significantly
- The antioxidant effect is associated with antagonism of the cellular effects of H₂O₂ (see for example extract Pa_f_Gli_20°C_28d 0.1%v/v)
- Stylopine shows no toxic (cytotoxic and irritant) effects following exposure for 24 hours at concentrations less than or equal to 10 µM. Furthermore, it has been surprisingly found that it may be used as a skin revitalizing, anti-fibrotic, cytoprotective and wound-healing agent (1 µM).
- Canadine shows no toxic (cytotoxic and irritant) effects following exposure for 24 hours at concentrations less than or equal to 1 µM. Furthermore, it has been surprisingly found that it may be used as a skin revitalizing, anti-fibrotic, cytoprotective and wound-healing agent.
- Protopine shows no toxic (cytotoxic and irritant) effects following exposure for 24 hours at concentrations less than or equal to 10 µM. Furthermore, it has been surprisingly found that it may be used as an anti-fibrotic, cytoprotective and wound-healing agent.
- Berberine shows no toxic (cytotoxic and irritant) effects following exposure for 24 hours at concentrations less than or equal to 10 µM. Furthermore, it has been surprisingly found that it may be used as a skin revitalizing and anti-fibrotic agent.
- Dihydroberberine displays toxic (irritant) effects following exposure for 24 hours at concentrations greater than or equal to 1 µM, which impair its potential use as an anti-radical, skin revitalizing and antifibrotic agent at that dosing regimen.
- Coptisine shows toxic (irritant) effects following exposure for 24 hours at concentrations greater than or equal to 1 µM.
- Chelerythrine shows no toxic (cytotoxic and irritant) effects following exposure for 24 hours at concentrations less than or equal to 1 µM. Furthermore, it has been surprisingly found that it may be used as a skin revitalizing, anti-fibrotic, cytoprotective and wound-healing agent.
- Sanguinarine shows toxic (cytotoxic and irritant) effects following exposure for 24 hours at concentrations greater than or equal to 1 µM.
- Chelidonine shows no toxic (cytotoxic and irritant) effects following exposure for 24 hours at concentrations less than or equal to 1 µM. Furthermore, it has been surprisingly found that it may be used as an anti-fibrotic, cytoprotective and wound-healing agent.
- The extracts Pa_f_Gli_20°C_28d and Fi_f_Gli_20°C_21d show no toxic (cytotoxic and irritant) effects following exposure for 24 hours at concentrations less than or equal to 0.1%. Furthermore, it has been surprisingly found that they may be used as anti-radical, skin revitalizing, anti-fibrotic and cytoprotective agents.
- A *C. majus* extract, to be used for a given medical or cosmetic application, should contain its constituents (including the isoquinoline alkaloids and the anti-radical components) in such ratios that the therapeutic concentrations of some constituents are not negatively affected by the toxic concentrations of one or more other constituents, in order to avoid that the whole positive effects are lost. For example, it would not be useful to use stylopine (for example 10 µM) in association with sanguinarine (for example 1 µM) in a ratio of 10:1 since the cytotoxic effects of the sanguinarine might mask the therapeutic effects (antifibrotic for example) of the stylopine.
- An optimal extract to be used as anti-radical, skin revitalizing, antifibrotic, cytoprotective and wound-healing agent might contain the following ratios of alkaloids:

| | |
|---|---|
| **Protopine** | **10 µM** |
| **Stylopine** | **1-10 µM** |
| **Chelidonine** | **1 µM** |
| **Coptisine** | **< 1 µM** |
| **Sanguinarine** | **< 0.1 µM** |
| **Berberine** | **1-10 µM** |
| **Chelerythrine** | **1 µM** |

in addition **to the anti-radical constituents.**

Therefore, the technical problem to be solved for obtaining an extract, endowed with the wanted properties, is lowering the concentration of sanguinarine, which is the most cytotoxic and irritant of the *C. majus* alkaloids, as much as possible.

Thanks to the preparation of a high number of extracts under different conditions and the analysis of their compositions, the authors have surprisingly found that sanguinarine is present in the nervation, stem, roots and in the fruit pericarp of *C. majus,* but is only present in very small quantities in the denervated leaves and in the flowers of the plant. Therefore, thanks to the research they have conducted, the authors have surprisingly found that for the preparation of an extract with the desired ratio between alkaloids, it is necessary to discard the nervation, stem, roots and the pericarp or only use the flowers and/or young leaves without the nervation.

Furthermore, the preferred solvents are water, propylene glycol, glycerine and mixtures thereof, as they allow the extraction of *C. majus* alkaloids in the desired ratios, and therefore are not toxic for dermatological use.

In conclusion, the results of this research have surprisingly lead the authors to identify novel uses in the fields of medicine and cosmetics for the substances of the invention. Said uses in the fields of medicine and cosmetics or the toxic effects of the substances of the invention at a given dosage, for 24 hours, are summarized in Table 1 below.

**Table 1.**

| **Treatment** | | **Use in the fields of medicine and cosmetics** | | | | | **Toxic effects** | |
|---|---|---|---|---|---|---|---|---|
| **Substance** | **Dose** | **Anti-radical** (skin protective) | **Revitalizing agent** | **Anti-fibrotic** (prevention of scar formation, also from acne) | **Cytoprotective** (antioxidant, anti-inflammatory and anti-ageing; useful also in the treatment of acne) | **Wound-healing** | **Cytotoxic** | **Irritant** |
| Berberine | 10 µm | No | Yes | Yes (↓collagen, ↓IL-6) | Yes* (↓IL-6) | No | No | No |
| | 1 µm | No | Yes | Yes (↓collagen, ↓IL-6) | Yes* (↓IL-6) | No | No | No |
| Dihydroberberine | 7.3 µm | Yes | Yes | Yes (↓collagen) | No | No | No | Yes (↑IL-6, ↑IL-1β) |
| | 1 µm | Yes | Yes | Yes (↓collagen) | No | No | No | Yes (↑IL-6, ↑IL-1β) |
| Coptisine | 10 µm | No | No | No | No | No | No | Yes (↑IL-6, ↑IL-1β) |
| | 1 pm | No | No | No | No | No | No | Yes (↑IL-6, ↑IL-1β) |
| Stylopine | 10 µm | No | Yes | Yes (↓collagen, ↓IL-6. ↓IL-1β) | Yes (↓IL-6, ↓IL-1β) | No | No | No |
| | 1 µm | No | Yes** | Yes (↓collagen, ↓IL-6, ↓IL-1β) | Yes (↓IL-6, ↓IL-1β) | Yes (↑profil) | No | No |
| Canadine | 10 µm | No | Yes | No | No | No | No | Yes (↓IL-1β) |
| | 1 µm | No | Yes | Yes (↓collagen, ↓IL-6, ↓IL-1β) | Yes (↓IL-6, ↓IL-1β) | Yes (↓IL-1β) | No | No |
| Chelerythrine | 10 µm | No | No | No | No | No | Yes (cell death, ↓dehydrogenase) | Yes (↑IL-6, ↑IL-1β) |
| | 1 µm | No | Yes | Yes (↓collagen, ↓IL-6, ↓IL-1β) | Yes (↓IL-6, ↓IL-1β) | Yes (↑prolif, ↓IL-1β) | No | No |
| Sanguinarine | 10 µm | No | No | No | No | No | Yes (cell death, ↓dehydrogenase) | Yes (↑IL-6, ↑IL-1β) |
| | 1 µm | No | No | No | No | No | Yes (senescent morph., ↓dehydrogenase) | Yes (↑IL-6, ↑IL-1β) |
| Chelidonine | 10 µm | No | No | No | No | No | Yes (senescent morph., ↓dehydrogenase) | Yes (↑IL-6, ↑IL-1β) |
| | 1 µm | No | No | Yes (↓collagen, ↓IL-6, ↓IL-1β) | Yes (↓IL-6, ↓IL-1β) | Yes (↓IL-1β) | No | No |
| Protopine | 10 µm | No | No | Yes (↓collagen, ↓IL-6, ↓IL-1β) | Yes (↓IL-6, ↓IL-1β) | Yes (↑prolif, ↓IL-1β) | No | No |
| Pa_f_Gli_20°C_28d | 0.1%. | Yes | Yes | Yes (↓collagen, ↓IL-6, ↓IL-1β) | Yes (↓IL-6, ↓IL-1β) | No | No | No |
| Fi_f_Gli_20°C_21d | 0.1%. | Yes | Yes | Yes (↓collagen, ↓IL-6, ↓IL-1β) | Yes (↓IL-6, ↓IL-1β) | No | No | No |

Another aspect of the invention concerns the use of some selected isoquinoline alkaloids selected from the group consisting of stylopine, canadine, dihydroberberine, protopine and tetrahydrocoptisine for the treatment of skin regeneration or repair disorders.

A further aspect of the invention concerns a cosmetic composition comprising the *Chelidonium majus* extract according to the invention and a cosmetically acceptable excipient.

A further aspect of the invention concerns a pharmaceutical composition comprising the *Chelidonium majus* extract according to the invention and a pharmaceutically acceptable excipient.

An additional aspect of the invention concerns a process for the preparation of a *Chelidonium majus* extract according to the present invention, said process comprising the following steps:
a. bringing the aerial parts selected from the group consisting of flowers, flower-buds, buds and leaves without nervation of the *Chelidonium majus* plant into contact with an extraction solvent, selected from the group consisting of propylene glycol, and aqueous solutions of HCl, at a temperature from 20°C to 50°C,
b. recovering the extract from the contact step,
   wherein the Chelidonium majus extract comprising protopine, stylopine, chelidonine, sanguinarine, chelerythrine, berberine, coptisine, wherein:
   the ratio between the quantity expressed in weight of stylopine with respect to the sum of the quantities expressed in weight of chelidonine, sanguinarine and chelerythrine is greater than or equal to 5;
   the ratio between the quantity expressed in weight of berberine with respect to the sum of the quantities expressed in weight of chelidonine, sanguinarine and chelerythrine is greater than or equal to 1; and;
   the ratio between the quantity expressed in weight of berberine with respect to the quantity expressed in weight of coptisine is greater than or equal to 0.04.

Indeed, it has also been surprisingly found that the isoquinoline alkaloids protopine, sanguinarine, chelerythrine, chelidonine, berberine, stylopine and coptisine, constituting the major isoquinoline alkaloids found in *Chelidonium majus,* are distributed in different ways in the various organs of said plant, and that their extraction is influenced by the extraction conditions. Thus, it is possible to obtain *C. majus* extracts that are particularly rich in the desired isoquinoline alkaloids (i.e. those that stimulate mitochondrial dehydrogenase activity) and, at the same time, are particularly depleted in the undesired ones (i.e. the alkaloids that inhibit mitochondrial dehydrogenase activity), just by changing the extraction conditions and/or by selecting the parts of the plant to be subjected to extraction.

Under an even further aspect, the invention concerns the *Chelidonium majus* extract according to the invention for use in the treatment of skin regeneration or repair dysfunctions.

Another aspect of the present invention concerns isoquinoline alkaloids selected from the group consisting of stylopine, canadine, dihydroberberine and tetrahydrocoptisine for use in the treatment of skin regeneration or repair disorders.

### Brief description of the figures

The characteristics and the advantages of the present invention will be evident from the detailed description reported below, and from the example embodiments provided by way of non-limiting illustration, in addition to the attached Figures, wherein:
- Figure 1 shows the effect of isoquinoline alkaloids on mitochondrial dehydrogenase activity in human dermal fibroblasts (HDF). % proliferation (Y axis) is reported versus concentrations of the substances (X axis), and, in particular:
   Figure 1a shows the effect of Protopine at times of 18h and 42h;
   Figure 1b shows the effect of Chelerythrine at times of 18h and 42h;
   Figure 1c shows the effect of Sanguinarine at times of 18h and 42h;
   Figure 1d shows the effect of Chelidonine at times of 18h and 42h;
   Figure 1e shows the effect of Berberine at times of 18h and 42h;
   Figure 1f shows the effect of Dihydroberberine at times of 18h and 42h;
   Figure 1g shows the effect of Canadine at times of 18h and 42h;
   Figure 1h shows the effect of Stylopine at times of 18h and 42h;
   Figure 1i shows the effect of Coptisine at times of 18h and 42h;
   Figure 1l shows the effect of Dihydroberberine, Canadine and Stylopine at 46h of incubation.
- Figure 2 shows the effect of isoquinoline alkaloids on mitochondrial dehydrogenase activity in human epidermal keratinocytes (HEK). Dose/response curves have been plotted at 6 concentrations. Tests have been performed at 24 hours, seeding 8,000 cells per well. % proliferation (Y axis) is reported versus concentrations of the substances (X axis), and, in particular:
   Figure 2a shows the effect of Protopine after 24h of incubation;
   Figure 2b shows the effect of Chelerythrine after 24h of incubation;
   Figure 2c shows the effect of Sanguinarine after 24h of incubation;
   Figure 2d shows the effect of Chelidonine after 24h of incubation; and
   Figure 2e shows the effect of Berberine after 24h of incubation.
- Figure 3 shows the effect of 4 extracts according to the invention on mitochondrial dehydrogenase activity in HDF cells. In particular, dose/response curves are shown at 6 concentrations of the extracts Fi_f_Gli_20°C_21d, Fi_f_Aq_45°C_24h, Fi_s_Aq_45°C_24h and Fi_f_HCl_20°C_21d.
- Figures 4 a - d show micro-photographic images at various magnifications of HDF cells stained for collagen using Sirius Red and subjected to various treatments with the compounds under test at specified concentrations, for a period of 24 hours, under standard growth conditions.
- Figures 5 a - d show micro-photographic images at various magnifications of HDF cells stained for collagen using Sirius Red and subjected to various treatments with the compounds under test at specified concentrations, for a period of 24 hours in the presence of 100 µM H₂O₂.
- Figures 6 a - f show micro-photographic images at various magnifications of HDF cells stained for collagen using Sirius Red and subjected to various treatments with the compounds under test at specified concentrations, for a period of 24 hours, in the presence of 34 ng/ml TGF-β1.
- Figure 7 shows the dose-antiradical activity curves. Concentration expressed as Log M indicates the base 10 logarithm of the concentration of the test substance in molarity, for example -5 corresponds to a concentration of 10 µM. Concentration expressed as Log %V/V indicates the base 10 logarithm of the concentration of the volume/volume percentage fraction, for example -2 corresponds to a concentration of 1%v/v.

### Detailed description of the invention

The invention thus concerns *Chelidonium majus* extracts comprising protopine, stylopine, chelidonine, sanguinarine, chelerythrine, berberine, coptisine.

Since these extracts are aimed at use in the field of cosmetics and in the medical-pharmaceutical field, said extracts must be safe from the clinical aspect, and able to be used to treat various skin conditions.

To this end, the extracts have a precise composition, and in particular an extract of *Chelidonium majus* has been identified comprising stylopine, chelidonine,
- the ratio between the quantity expressed in weight of stylopine with respect to the sum of the quantities expressed in weight of chelidonine, sanguinarine and chelerythrine is greater than or equal to 5;
- the ratio between the quantity expressed in weight of berberine with respect to the sum of the quantities expressed in weight of chelidonine, sanguinarine and chelerythrine is greater than or equal to 1; and;
- the ratio between the quantity expressed in weight of berberine with respect to the quantity expressed in weight of coptisine is greater than or equal to 0.04.

For the purposes of the present invention:
- the term *"Chelidonium majus"* or *"C. majus"* or "Celidonia" means a perennial herbaceous plant from the Mediterranean area, belonging to the genus *Chelidonium* and to the Papaveraceae family, characterised by a yellow-orange latex that oxidises and darkens rapidly on exposure to air and that leaks from the branches once broken. It is understood that the invention comprises all varieties and sub-species of said plant, such as *Chelidonium majus* var. *majus* and *Chelidonium majus* var. *laciniatum.*
- the term "areal parts of the *"Chelidonium majus"* plant means the plant organs that develop out of the soil under natural conditions, such as flowers, flower-buds, buds and leaves.

Advantageously, the *Chelidonium majus* extract according to the invention is an extract wherein:
- the ratio between the quantity expressed in weight of stylopine with respect to the sum of the quantities expressed in weight of chelidonine, sanguinarine and chelerythrine is greater than or equal to 5;
- the ratio between the quantity expressed in weight of berberine with respect to the sum of the quantities expressed in weight of chelidonine, sanguinarine and chelerythrine is greater than or equal to 5; and;
- the ratio between the quantity expressed in weight of berberine with respect to the quantity expressed in weight of coptisine is greater than or equal to 0.04. Even more preferable, it is the *Chelidonium majus* extract according to the invention, wherein:
- the ratio between the quantity expressed in weight of stylopine with respect to the sum of the quantities expressed in weight of chelidonine, sanguinarine and chelerythrine is greater than or equal to 50;
- the ratio between the quantity expressed in weight of berberine with respect to the sum of the quantities expressed in weight of chelidonine, sanguinarine and chelerythrine is greater than or equal to 10; and;
- the ratio between the quantity expressed in weight of berberine with respect to the quantity expressed in weight of coptisine is greater than or equal to 0.05.

The *Chelidonium majus* extracts according to the invention are surprisingly useful for treating skin regeneration or repair dysfunctions.

Skin is understood to mean a covering tissue, not limited to the skin, but may also include other epithelial and connective tissues such as gum tissue, oral mucosa and nasal mucosa.

The term skin regeneration or repair dysfunctions refers to states of irritation and/or inflammation of the skin such as reddening, states associated with skin ageing, wrinkles, wounds, sores, ulcers, burns and skin lesions in general, fibrosis and fibrotic conditions, the results of scarring in general, scars, hypertrophic scars, atrophic scars, stretch marks, acne, dermatous lesions and eczema.

Under another aspect, the invention concerns a cosmetic composition comprising the *Chelidonium majus* extract according to the invention and a cosmetically acceptable excipient.

In certain embodiments, the cosmetically acceptable carrier for the composition of the invention is an excipient, carrier or diluent suitable for cosmetic formulations. Under a further aspect, the invention concerns a pharmaceutical composition comprising the *Chelidonium majus* extract according to the invention and a pharmaceutically acceptable excipient.

In certain embodiments, the pharmaceutically acceptable carrier for the composition of the invention is an excipient, carrier or diluent suitable for pharmaceutical formulations.

The compositions may be formulated, for example, in gels, lotions, foams, sticks, ointments, milks or creams.

In certain embodiments, the composition comprising the extract according to the invention is applied to the skin requiring treatment.

Under yet another aspect, the invention concerns a process for the preparation of a *Chelidonium majus* extract according to the present invention, said process comprising the following steps:
a. bringing the aerial parts selected from the group consisting of flowers, flower-buds, buds and leaves without nervation of the *Chelidonium majus* plant into contact with an extraction solvent, selected from the group consisting of propylene glycol, and aqueous solutions of HCl, at a temperature from 20°C to 50°C,
b. recovering the extract from the contact step,
   wherein the Chelidonium majus extract comprising protopine, stylopine, chelidonine, sanguinarine, chelerythrine, berberine, coptisine, wherein:
   the ratio between the quantity expressed in weight of stylopine with respect to the sum of the quantities expressed in weight of chelidonine, sanguinarine and chelerythrine is greater than or equal to 5;
   the ratio between the quantity expressed in weight of berberine with respect to the sum of the quantities expressed in weight of chelidonine, sanguinarine and chelerythrine is greater than or equal to 1; and;
   the ratio between the quantity expressed in weight of berberine with respect to the quantity expressed in weight of coptisine is greater than or equal to 0.04.

Under an even further aspect, the invention concerns the *Chelidonium majus* extract obtainable by the process of claim 1 for use in skin regeneration or repair dysfunctions.

In step a. of the process according to the invention, said aerial parts of the *Chelidonium majus* plant are selected from the group consisting of flowers, flower-buds, buds and leaves, preferably leaves without the nervation. Even though said areal parts of the C. *majus* plant may be used fresh in the extraction process, it is possible to subject them to a stabilization or preservation treatment such as freezing, freeze drying or drying prior to said process. Said treatment has the advantage of allowing preservation of the plant with the scope of using it even after harvesting, without experiencing loss or degradation of the active substances contained therein. Said areal parts of the *C. majus* plant may also be subjected to crushing, for example by cutting to a size appropriate for infusion. Said crushing process advantageously increases the extraction solvent contact surface, making the extraction process itself more effective.

The extraction solvent of step a. is selected from the group consisting of propylene glycol, glycerol, water, including demineralised water, distilled water, deionised water, mineral water, thermal water, spring water and sea water, acidic aqueous solutions, comprising aqueous solutions of HCI and aqueous solutions of citric acid and wherein said step a. is carried out at a temperature between 0°C and the boiling point temperature of the solvent, preferably from 20°C to 50°C.

Said contact step a. is advantageously performed using quantities of *Chelidonium majus* of between 10 g and 350 g, preferably between 30 g and 200 g per 1000 ml of extraction solvent.

Advantageously, the extraction of said process may be performed by means of any method known in the art, in particular by means of extraction by maceration, extraction by infusion, extraction by digestion or extraction by percolation or combinations of said techniques and may be performed under various conditions in terms of temperature, pressure and time.

Preferably, the recovery of the extract of said step b. may be performed by means of any method known in the art, in particular by means of filtration, decantation, pressing, hydraulic pressing, centrifugation or combinations of said techniques. Even though the *Chelidonium majus* extract according to the invention may be used as it is, it is also possible to subject it to a post-treatment step, such as dilution, concentration, sterilization, further extraction, isolation of one or more components such as stylopine, supplemented with substances of various types, for example supplemented with additional active substances.

The *Chelidonium majus* extract according to the invention may furthermore be packaged in a form suitable for distribution.

Under an even further aspect, the invention concerns the *Chelidonium majus* extract according to the invention for use in the treatment of skin regeneration or repair dysfunctions.

Another aspect of the present invention concerns isoquinoline alkaloids selected from the group consisting of stylopine, canadine, dihydroberberine, protopine and tetrahydrocoptisine for use in the treatment of skin regeneration or repair disorders.

Example embodiments of the present invention, provided by way of illustration, are reported below.

### EXAMPLES

### EXAMPLE 1: PREPARATION AND CHEMICAL CHARACTERIZATION OF THE EXTRACTS

### Finding, harvesting and preparation of the parts of the plant for extraction

The plant used comes from the Garfagnana area (Lucca, Italy) or from Sardinia (Sassari and Olbia, Italy) or from the Irpinia mountains (Rome, Italy) with regard to spontaneously growing plants, or from the hills around Pisa (San Giuliano Terme, Pisa, Italy) with regard to cultivated plants. Alternatively, the plants were in the form of cut and dried areal parts from Hungary, distributed by Aboca spa (Arezzo, Italy).

In the case of the use of fresh plants, only the areal parts of the plants have been harvested between April to July, and said areal parts have been cut up or separated into their organs such as flowers, flower buds, buds and leaves devoid of the main nervation, etc. and these have been cut up and used for preparation of the extracts as they are or frozen or sun-dried for 7 days or oven-dried at 70°C for 8 hours or at 30°C for 3 days.

### Extraction by maceration

Only the areal parts of the plant have been used for production of the macerates: flowers, flower-buds, buds, leaves with the main nervation removed in fresh, frozen, dried form or treated in another manner which may be subdivided into smaller portions. The plant parts are placed in a container in the presence of a quantity of extraction solvent at a temperature that varies, preferably between 0°C and room temperature, generally 20°C, for a certain variable period of time, generally between 7 days and 28 days. After said period of time, the extract is recovered by means of decanting off the solvent, pressing of the natural substance and filtration of the extract with the aim of removing any particulate materials.

### Extraction by infusion

Only the areal parts of the plant are used for the production of infusions: flowers, flower-buds, buds, leaves with the main nervation removed in fresh, frozen, dried form, or treated in another manner that may be subdivided into smaller portions, are placed in a container and the extraction solvent poured in at boiling point and then all left for a certain period of time, varying in general between 1 minute to 1 hour. After said period of time, the extract is recovered by means of decanting off the solvent, pressing of the natural substance and filtration of the extract with the aim of removing any particulate materials.

### Extraction by digestion

Only the areal parts of the plant are used for the production of digests: flowers, flower-buds, buds, leaves with the main nervation removed in fresh frozen or dried form or treated in another manner that may be subdivided into smaller portions, are placed in a container in the presence of a quantity of extraction solvent at a temperature varying between room temperature, generally 20°C, and the boiling point of the solvent for a certain variable period of time, generally between 24 and 72 hours. After said period of time, the extract is recovered by means of decanting off the solvent, pressing of the natural substance and filtration of the extract with the aim of removing any particulate materials.

### Extraction by percolation

Only the areal parts of the plant are used for the production of percolates: flowers, flower-buds, buds, leaves with the main nervation removed in fresh frozen or dried form or treated in another manner that may be subdivided into smaller portions, are placed in a percolator in the presence of a quantity of extraction solvent at a temperature varying between the boiling point of the solvent and the freezing point. Extraction solvent is then passed through the natural substance in the percolator It is possible for there to be one or more cycles in which the extraction solvent passes through the natural substance for a certain variable period of time, generally between 1 minute to 24 hours. After said period of time, the extract is recovered by means of decanting off the solvent, pressing of the natural substance and filtration of the extract with the aim of removing any particulate materials.

### HPLC analysis aimed at estimating the concentrations of isoquinoline alkaloids in C. maius extracts

Titration was performed by HPLC according to an adaptation of the method described in: Sarkozi A et al. Chromatographia. 2006, 63, S81-S86. Said adaptation has also allowed the identification of the alkaloid stylopine.

In detail, the analyses have been performed using a Perkin-Elmer Flexar HPLC consisting of a binary pump, a three channel degasser and a UV/VIS detector. The TotalChrome software program was used for data acquisition and processing. Separation was conducted on a Phenomenex Luna 5 µl C18 reverse phase column (250 mm x 4.6 mm I.D.). The wavelength used was 280 nm. The injection volume was 20 µl (loop size). An isocratic mixture of acetonitrile-methanol-30mM ammonium formate (pH 2.8) 150:180:670 (v/v/v) has been used as eluent. The flow rate was 0.8 ml/min. Identification of the components was achieved by the addition of specific standard samples in the amount of 5 µl in 35 µl of sample. The *C. majus* extracts are characterised by the concentrations of the major alkaloids, as reported in Table 2.

In the following tables each extract is named, on the basis of the extraction conditions, through a label generated according to what is summarized below.

Each label consists of fields separated by the symbol_wherein each field refers to a characteristic of the manner by which the extract has been prepared. In detail, PA = areal parts, Fi = flowers, Bo = flower buds, Fo-ner = leaves depleted of nervation, Fr = fruit, Fo = leaves, RP = primary roots, RS = secondary roots, St = stem, CA = stalk, Ner = nervation, f = fresh, c = frozen, s = dried, Gli = propylene glycol; Aq = water, pH2 = aqueous citric acid solution at pH 2, HCl = aqueous solution of 5% HCI, Glic = glycerol, EtOH = ethanol, "number"°C = extraction temperature, "number"h = hours of extraction and "number"d = days of extraction.

**Table 2. C. majus extracts and their method of preparation**

| **Extract** | **No.** | **Plant part** | **Plant origin** | **Fresh, dried or frozen plant** | **Extraction solvent** | **Ratio plant/solvent %w/v** | **Extraction temperature** | **Extraction time** | **Method of extraction** | **Extract of the invention or Reference extract** |
|---|---|---|---|---|---|---|---|---|---|---|
| PA_f_Gli_20°C_28d | 1 | Areal parts | Ernici mountains | Fresh | Propylene glycol | 20 | Room Temp. | 28 days | Maceration | Invention |
| Fi_f_HCl_20°C_21d | 2 | flowers | Garfagnana | Fresh | 5% HCl | 16 | Room Temp. | 21 days | Maceration | Invention |
| Fi_f_HCl_20°C_7d | 3 | flowers | Garfagnana | Fresh | 5 % HCl | 17 | Room Temp. | 7 days | Maceration | Invention |
| Fi_f_Gli_20°C_21d | 4 | flowers | Ernici mountains | Fresh | Propylene glycol | 20 | Room Temp. | 21 days | Maceration | Invention |
| Fi_f_Aq_45°C_24h | 5 | flowers | Gallura | Frozen | Water | 20 | 45°C. | 24 hours | Digestion | |
| Fi_s_Aq_45°C_24h | 6 | flowers | Pisan hills | Dried at 30°C for 3 days | Uliveto mineral water, pH 5.8 | 20 | 45°C. | 24 hours | Digestion | |
| Fo-ner_f_Aq_45°C_24h | 7 | leaves devoid of main nervation | Pisan hills | Fresh | Uliveto mineral water, pH 5.8 | 20 | 45°C. | 24 hours | Digestion | |
| Bo_f_Aq_45°C_24h | 8 | flower buds | Pisan hills | Fresh | Uliveto mineral water, pH 5.8 | 20 | 45°C. | 24 hours | Digestion | invention |
| Fi_s_pH2_50°C_24h | 9 | flowers | Garfagnana | Sun dried for 7 days | Citric acid pH 2 | 1 | 50°C. | 24 hours | Digestion | |
| Fi_f_pH2_50°C_72h | 10 | flowers | Garfagnana | Frozen | Citric acid pH 2 | 3 | 50°C. | 72 hours | Digestion | |

| **Extract** | **No.** | **Plant part** | **Plant origin** | **Fresh, dried or frozen Plant** | **Extraction solvent** | **Ratio plant/solvent %w/v** | **Extraction temperature** | **Extraction time** | **Method of extraction** | **Extract of the invention or Reference extract** |
|---|---|---|---|---|---|---|---|---|---|---|
| Fr_Aq_45°C_24h | 11 | Fruit | Pisan hills | Fresh | Water pH 7 | 20 | 45 | 24 hours | Digestion | Reference |
| Ner_f_Aq_45°C_24h | 12 | Nervation | Pisan hills | Fresh | Water pH 7 | 20 | 45 | 24 hours | Digestion | Reference |
| PA_s_HCl_20°C_21d | 13 | Areal parts | Hungary | Dried | Water pH 0 from 5% HCl | 25 | 20 | 21 days | Maceration | Reference |
| Fr_f_HCl_20°C_21d | 14 | Fruit | Garfagnana | Fresh | Water pH 0 from 5% HCl | 22 | 20 | 21 days | Maceration | Reference |
| PA_s_Glic_20°C_21d | 15 | Areal parts | Hungary | Dried | Glycerol | 20 | 20 | 21 days | Maceration | Reference |
| PA_s_Glic_50°C_72h | 16 | Areal parts | Hungary | Dried | Glycerol | 3 | 50 | 72 hours | Digestion | Reference |
| PA_c_EtOH_50°C_72h | 17 | Areal parts | Hungary | Frozen | Ethanol | 3 | 50 | 72 hours | Digestion | Reference |
| Fr_c_Aq_50°C_72h | 18 | Fruit | Garfagnana | Frozen | Water pH 7 | 3 | 50 | 72 hours | Digestion | Reference |
| Fo_f_HCl_20°C_21d | 19 | Leaves | Garfagnana | Fresh | Water pH 0 from 5% HCl | 26 | 20 | 21 days | Maceration | Reference |
| Ca_f_HCl_20°C_21d | 20 | Stalks | Garfagnana | Fresh | Water pH 0 from 5% HCl | 27 | 20 | 21 days | Maceration | Reference |
| RP_f_HCl_20°C_21d | 21 | Primary roots | Garfagnana | Fresh | Water pH 0 from 5% HCl | 75 | 20 | 21 days | Maceration | Reference |
| Ca_c_pH2_50°C_72h | 22 | Stalks | Garfagnana | Frozen | Water at pH 2 from citric acid | 3 | 50 | 72 hours | Digestion | Reference |

| **Extract** | **No.** | **Plant part** | **Plant origin** | **Fresh, dried or frozen plant** | **Extraction solvent** | **Ratio plant/solvent %w/v** | **Extraction temperature** | **Extraction time** | **Method of extraction** | **Extract of the invention or Reference extract** |
|---|---|---|---|---|---|---|---|---|---|---|
| Ca_c_Aq_50°C_72h | 23 | Stalks | Garfagnana | Frozen | Water pH 7 | 3 | 50 | 72 hours | Digestion | Reference |
| Fo_c_pH2_50°C_72h | 24 | Leaves | Garfagnana | Frozen | Water at pH 2 from citric acid | 3 | 50 | 72 hours | Digestion | Reference |
| Fr_c_pH2_50°C_72h | 25 | Fruit | Garfagnana | Frozen | Water at pH 2 from citric acid | 3 | 50 | 72 hours | Digestion | Reference |
| RP_c_pH2_50°C_72h | 26 | Primary roots | Garfagnana | Frozen | Water at pH 2 from citric acid | 3 | 50 | 72 hours | Digestion | Reference |
| RP_c_pH7_50°C_72h | 27 | Primary roots | Garfagnana | Frozen | Water pH 7 | 3 | 50 | 72 hours | Digestion | Reference |
| RS_c_pH2_50°C_72h | 28 | Secondary roots | Garfagnana | Frozen | Water at pH 2 from citric acid | 3 | 50 | 72 hours | Digestion | Reference |
| RS_c_Aq_50°C_72h | 29 | Secondary roots | Garfagnana | Frozen | Water pH 7 | 3 | 50 | 72 hours | Digestion | Reference |
| St_c_pH2_50°C_72h | 30 | Stem | Garfagnana | Frozen | Water at pH 2 from citric acid | 3 | 50 | 72 hours | Digestion | Reference |
| St_c_Aq_50°C_72h | 31 | Stem | Garfagnana | Frozen | Water pH 7 | 3 | 50 | 72 hours | Digestion | Reference |

Table 3 reports the compositions of the extracts obtained above, as determined by HPLC.

**Table 3. Extracts and quantities of the alkaloids, expressed as the logarithm of the molar concentration (logM), as measured by HPLC. The values are derived from the mean of three independent HPLC analyses and are reported to an accuracy of two decimal digits or in any case to the smallest significant digit.**

| **Extract** | **Protopine** | **Chelidonine** | **Coptisine** | **Stylopine** | **Sanguinarine** | **Berberine** | **Cheterythrine** | **Extract of the invention or Reference extract** |
|---|---|---|---|---|---|---|---|---|
| PA_f_Gli_20°C_28d | -5.08 | -4.80 | -3.17 | -3.47 | -8.03 | -4.16 | <-8 | Invention |
| Fi_s_pH2_50°C_24h | -7.18 | -7.28 | -4.00 | -4.48 | -7.67 | -5.21 | <-8 | Invention |
| Fi_f_Aq_45°C_24h | -4.63 | -6.24 | -3.53 | -5.00 | -8.31 | -4.41 | <-8 | Invention |
| Fi_s_Aq_45°C_24h | -4.13 | -5.24 | -2.69 | -3.86 | -6.49 | -3.76 | -7.17 | Invention |
| Fi_f_Gli_20°C_21d | -4.70 | -5.56 | -3.02 | -3.38 | -8.13 | -4.08 | <-8 | Invention |
| Bo_f_Aq_45°C_24h | -4.69 | -8.01 | -3.81 | -4.90 | -8.31 | -4.73 | <-8 | Invention |
| Fi_f_pH2_50°C_72h | -5.19 | -8.01 | -4.01 | -4.60 | -5.86 | -5.07 | <-8 | Invention |
| Fo-ner_f_Aq_45°C_24h | -4.77 | -5.09 | -3.42 | -3.60 | -8.03 | -3.87 | <-8 | Invention |
| Fi_f_HCl_20°C_21d | -4.52 | -5.81 | -4.17 | -4.03 | -5.24 | -4.34 | <-8 | Invention |
| Fr_f_Aq_45°C_24h | -4.41 | -4.86 | -3.62 | -6.75 | -7.56 | -4.49 | -7.37 | Reference |
| Ner_f_Aq_45°C_24h | -4.34 | -4.50 | -3.39 | -6.06 | -5.12 | -4.17 | <-8 | Reference |
| PA_s_HCl_20°C_21d | -4.19 | -4.48 | -3.63 | -3.71 | -4.53 | -5.27 | -5.70 | Reference |
| Fr_f_HCl_20°C_21d | -4.44 | -4.33 | -4.24 | -4.12 | -4.25 | -4.15 | -5.61 | Reference |
| PA_s_Glic_20°C_21d | -4.66 | -4.99 | -3.44 | -4.27 | -5.41 | -5.23 | <-8 | Reference |
| PA_s_Glic_50°C_72h | -4.83 | -4.90 | -3.57 | -3.80 | -5.93 | -5.32 | <-8 | Reference |
| PA_c_EtOH_50°C_72h | -4.75 | -5.42 | -3.97 | -4.37 | -5.18 | -5.59 | -6.46 | Reference |
| Fr_c_Aq_50°C_72h | -4.91 | -4.76 | -3.39 | -5.26 | -5.34 | -4.71 | <-8 | Reference |
| Fo_f_Aq_20°C_21d | -4.73 | -4.60 | -4.42 | -4.01 | -5.02 | -4.48 | -6.15 | Reference |
| Ca_f_Aq_20°C_21d | -4.83 | -4.47 | -4.63 | -4.75 | -5.44 | -4.48 | -6.12 | Reference |
| RP_f_Aq_20°C_21d | -4.12 | -3.41 | -4.09 | -4.52 | -3.87 | -4.53 | -4.31 | Reference |
| Ca_c_pH2_50°C_72h | -5.45 | -4.90 | -4.82 | -6.26 | -4.93 | -5.82 | -5.93 | Reference |
| Ca_c_Aq_50°C_72h | -5.40 | -5.07 | -5.12 | -8.17 | -6.23 | -6.27 | -6.77 | Reference |
| Fo_c_pH2_50°C_72h | -5.33 | -4.84 | -3.93 | -5.29 | -4.81 | -4.52 | -5.65 | Reference |
| Fr_c_pH2_50°C_72h | -4.94 | -4.63 | -3.42 | -4.58 | -4.27 | -4.52 | -5.56 | Reference |
| RP_c_pH2_50°C_72h | -4.97 | -4.07 | -4.70 | -5.57 | -4.06 | -5.43 | -4.87 | Reference |
| RP_c_pH7_50°C_72h | -4.88 | -4.19 | -4.87 | -6.06 | -5.46 | -5.74 | -5.88 | Reference |
| RS_c_pH2_50°C_72h | -4.62 | -3.65 | -4.12 | -5.05 | -3.70 | -5.08 | -4.33 | Reference |
| RS_c_Aq_50°C_72h | -4.78 | -3.92 | -4.40 | -8.17 | -5.03 | -5.36 | -5.39 | Reference |
| St_c_pH2_50°C_72h | -5.55 | -4.67 | -4.74 | -6.16 | -4.51 | -5.52 | -5.52 | Reference |
| St_c_Aq_50°C_72h | -5.76 | -5.06 | -5.03 | -8.17 | -8.31 | -8.06 | <-8 | Reference |

### EXAMPLE 2: BIOLOGICAL ASSAYS AND ANTI-RADICAL ACTIVITY

### CELL CULTURES AND TREATMENTS

Human dermal fibroblasts (HDF) have been cultured and handled in accordance with the suppliers instructions (ECACC, cat. 106-05a, http://www.hpacultures.org.uk/).

Human keratinocytes (HEK, Human Epidermal Keratinocytes, ECACC) have been cultured and handled in accordance with the suppliers instructions (ECACC, cat. 102-05a, http://www.hpacultures.org.uk/).

HUVEC cells (Human Umbilical Vein Endothelial Cells, ECACC) have been cultured and handled in accordance with the suppliers instructions (ECACC, cat. 200-05n, http://www.hpacultures.orq.uk/).

For what concerns the quantification of the inflammatory cytokines in the cell supernatant, analyzed by means of an ELISA method, the quantification of collagen produced by HDF cells, the evaluation of cell proliferation and the evaluation of cell morphology, the selected cells were seeded in flat-bottomed 24 well plates (Corning), allowed to adhere and then treated in triplicate with either the test substance at a given concentration, or with control medium in the presence or absence of 100 µM H₂O₂ or 34 ng/ml TGF-β1. The conditions under which the cells have been treated in triplicate with either the test substance at the desired concentration or with control medium are defined "standard growth conditions". The total volume of culture medium per well was 500 µl. Following a treatment period of 24 hours, the cell supernatant or the cells was analyzed as best described in the sections "Measurement of inflammatory cytokines in cell supernatant" and "Fixing the cells with methanol".

With regard to assays of mitochondrial dehydrogenase activity, HDF HEK and HUVEC cells were seeded in flat bottomed 96 well plates (Sarstedt), at a density of 2,000 to 8,000 cells per well, allowed to adhere and then treated in quadruplicate with test substance at the desired concentration or with control medium. The total volume of culture medium per well was 100 µl. In particular, the test substances were used within a concentration range going from 10⁻⁴ to 10⁻⁹ M, in the case of individual alkaloids, and within a concentration range going from 1%v/v to 0.0001%v/v, in the case of extracts. Human dermal fibroblasts were used for assay at the 3rd T75 passage. Human keratinocytes were used for assay between the 1st and 6th passage. HUVEC cells were used for assay between the 1st and 6th passage.

Following a treatment period of 18 hours or 42 hours, the cells were analyzed as best described in the section "Evaluation of mitochondrial dehydrogenase activity".

With regard to the assays aimed at evaluating cell migration, the fibroblast or HUVEC cells were cultured in flat-bottomed 96 well plates (Sarstedt), bringing the cells to confluence. The evaluation of the effects of the substance, at a concentration of 0.02% v/v, on the migration of fibroblasts or HUVEC cells, was performed as described in the section Evaluation of the modulation of cell migration.

### EVALUATION OF MITOCHONDRIAL DEHYDROGENASE ACTIVITY

Mitochondrial dehydrogenase activity was measured in HDF, HEK and HUVEC cells by means of the formazan assay, using the WST-1 kit from Roche Diagnostic in accordance with the supplier's instructions. Briefly: the reagent was added (10 µl per well), the incubation was carried out for a further 2 hours or 4 hours, and, at the end of this time, the absorbance was read for 1 s at a wavelength of 450 nm in a Perkin Elmer Victor 2 Wallac microplate reader.

The results are given in Figures 1 to 3, where mitochondrial dehydrogenase activity, expressed as activity percentage, is reported versus the concentration of test substance. The activity percentage (% mitochondrial dehydrogenase activity) is defined as: % mitochondrial dehydrogenase activity = ((absorbance of treated cells - absorbance of blank) / (absorbance of control - absorbance of blank)) x 100.

### MEASUREMENT OF INFLAMMATORY CYTOKINES IN CELL SUPERNATANT

The inflammatory cytokines, CD40 ligand (CD40L), interferon γ (INF-γ), interleukin 1α (IL-1α), interleukin 1β (IL-1β), interleukin 6 (IL-6), interleukin 8 (IL-8), interleukin 17 (IL-17) and tumour necrosis factor α (TNF-α) were determined qualitatively and quantitatively. Each measurement was performed in duplicate using a Mosaic™ ELISA protein *microarray* assay system from R&D System in accordance with the supplier's instructions. Furthermore, with the aim of obtaining a correlation model between light signal intensity and cytokine concentration, a calibration curve using 6 concentrations was performed for each of them. Analysis of the data was performed using the dedicated Q-View™ Imager software package from Quansys Biosciences

### FIXING THE CELLS WITH METHANOL

After the cell supernatant was removed, the HDF cells were fixed overnight using cold methanol at -20°C.

### SIRIUS RED CELL STAINING PROTOCOL FOR COLLAGEN

Following overnight fixing with methanol, the solvent was removed and the fixed HDF cells were stained with 400 µl/well of a 0.1% solution of Picro-Sirius Red (PSR) for 4 hours. Said solution is prepared by dissolving the reagent "Direct Red 80" from Sigma-Aldrich in a saturated aqueous solution of picric acid. Following 4 hours of incubation with PSR at room temperature, the cells were washed twice with 200 µl/well of a 0.1% aqueous solution of acetic acid and dried.

### EVALUATION OF CELL PROLIFERATION

Photographs of the HDF cells stained with Sirius Red in all the wells of the plates were taken using a microscope at 100x magnification and their number, for each photograph, has been evaluated by using the "cell counting" function of the ImageJ software program.

### EVALUATION OF CELL MORPHOLOGY

Moreover, photographs of the HDF cells stained with Sirius Red were taken using a microscope at 100x, 200x and 400x magnification. Their morphology, colour and shape have been evaluated by using the ImageJ software program.

### MEASUREMENT OF THE QUANTITY OF COLLAGEN PRODUCED BY THE CELLS BY MEANS OF ELUTION OF THE SIRIUS RED

The HDF cells stained with Sirius Red were subjected to elution of the stain in a manner analogous to the one reported in "Am. J. Physiol. Renal. Physiol., 2007". Briefly, the stain trapped within the cells was eluted with 200 µl/well of a 0.1 N aqueous solution of sodium hydroxide by shaking in a tilting shaker for 2 hours, then samples of the eluted stain solution was transferred into 96 well plates. The optical density (OD) was read using a Wallac microplate reader in light absorbance at a wavelength of 530 nm.

### Evaluation of the modulation of cell migration

This assay was performed in order to assess the effects of the *Chelidonium majus* extracts at a concentration of 0.02% v/v on the migration of fibroblasts and HUVEC cells.

Once at confluence, an artificial cross-shaped wound was made on the cell monolayer for each sample using a sterile plastic tip. The cell culture medium was then removed and replaced by a medium containing the test substance, except for the negative controls.

Immediately after execution of the wound (t = 0 h) and at various times (t = 24 and 48 h), phase contrast microscope observations of the treated samples and the controls were made using an inverted phase contrast microscope (INV-2 Orma) at a magnification going from 200X to 400X. The results are reported as the areas of the observation zone contiguous to the wound where the presence of cells is observed. An increase in said area at increasing times corresponds to a reduction in the dimensions of the wound, indicating that cells are migrating towards the area inside the cut. The measurement of said area has been performed using the CellProfiler software program [Carpenter AE et al. Genome Biology, 2006, 7, R100].

### EVALUATION OF ANTI-RADICAL ACTIVITY

With regard to assays aimed at evaluating anti-radical activity, a chemical test was used where the decay of the diphenylpicrylhydrazyl (DPPH) radical is assessed in the absence or in the presence of the test substances. Indeed, the DPPH radical is used to test the ability of substances to act as free radical scavengers. DPPH in solution has a purple colour with a maximum absorbance peak at 517 nm which changes to yellow-colourless when this radical extracts a hydrogen atom from a radical scavenger to give rise to the reduced form DPPH-H. The assay has been conducted in quadruplicate in 96 well microplates (SARSTEDT Tissue Culture Plate 96-well flat bottom cell+). 50 µl of an ethanol solution of the test substance at 2X concentration and then 50 µl of a 200 µM solution of DPPH (MW 168,18, Aldrich 2,2-di(4-tert-octylphenyl)-1-picrylhydrazyl, free radical Lot# MKBF7574V Cat N° 257621-100 mg) have been added to each well of the microplate. A positive control consisting of 0.1 mM vitamin C and a negative control consisting of ethanol alone were included in each microplate. After 30 minutes from the addition of DPPH, photographs of the plates were taken (Canon PowerShot A610 5.0 Mpixels) and read spectrophotometrically at 530 nm.

### STATISTICAL ANALYSIS

The cell proliferation, collagen production and inflammatory cytokine production values obtained were subjected to statistical analysis using the OpenStat software program, version 26.03.2012 (www.statprograms4u.com). In particular, the comparison between mean values was performed using a two tail Student's *t*-test. Values of P≤0.05 were considered statistically significant.

### RESULTS OF THE BIOLOGICAL ASSAYS AND ANTI-RADICAL ACTIVITY

The results of assays aimed at evaluating the effects of the substances on mitochondrial dehydrogenase activity on HDF cells are reported in Figures 1a - 1l, wherein dose/response curves are plotted at 6 concentrations for all compounds analyzed. The tests were performed with observations after 18 hours, seeding 8,000 cells per well, and after 42 hours, seeding 2,000 cells per well. Furthermore, in order to better analyze the dose/response curves for dihydroberberine, canadine and stylopine, curves were collected at 11 concentrations by seeding 2,000 cells per well with 46 hours incubation.

The results of assays aimed at evaluating the effect on mitochondrial dehydrogenase activity on HEK cells are reported in Figures 2a - 2e. Dose/response curves are plotted at 6 concentrations. The tests were performed with observations after 24 hours, seeding 8,000 cells per well.

The results of assays aimed at evaluating the effects of the Fi_f_Gli20°C_21d, Fi_f_Aq_45°C_24h, Fi_s_Aq_45°C_24h and Fi_f_HCl_20°C_21d extracts on mitochondrial dehydrogenase activity on HDF cells are reported in Figure 3. Dose/response curves are plotted at 6 concentrations. The tests were performed with observations after 24 hours, seeding 8,000 cells per well.

The results of assays aimed at evaluating the. effect of *C. majus* alkaloids and extracts on mitochondrial dehydrogenase activity are reported in Table 4.

**Table 4. Results obtained for mitochondrial dehydrogenase activity.**

| **TREATMENT** | **% mitochondrial dehydrogenase activity ± SD** | | | |
|---|---|---|---|---|
| | **18 h** | **24 h** | **42 h** | **46 h** |
| **Control (C)** | 100.00 ± 8.69 | 100.00 ± 7.52 | 100.00 ± 10.26 | 100.00 ± 7.98 |
| **10 µM berberine** | 114.10 ± 10.04 | - | 132.23 ± 10.22 | - |
| **1 µM berberine** | 113.51 ± 2.98 | - | 140.10 ± 8.89 | - |
| **7.3 µM dihydroberberine** | 127.92 ± 13.77 | - | 137.32 ± 17.63 | 192.19 ± 24.35 |
| **1 µM dihydroberberine** | 131.38 ± 5.40 | - | 143.07 ± 14.09 | 120.89 ± 19.61 |
| **10 µM coptisine** | - | 62.33 ± 11.82 | 139.12 ± 5.34 | - |
| **1 µM coptisine** | - | 84.53 ± 3.17 | 90.09 ± 2.30 | - |
| **10 µM stylopine** | 107.11 ± 3.00 | - | 150.96 ± 12.83 | 138.07 ± 23.95 |
| **1 µM stylopine** | 104.98 ± 9.50 | - | 100.39 ± 15.84 | 95.66 11.40 |
| **10 µM canadine** | 129.96 ± 32.44 | - | 124.25 ± 5.34 | 129.80 ± 11.10 |
| **1 µM canadine** | 126.82 ± 3.64 | - | 100.52 ± 10.59 | 106.15 ± 5.25 |
| **10 µM chelidonine** | 89.33 ± 6.90 | - | 30.30 ± 2.97 | - |
| **1 µM chelidonine** | 105.47 ± 7.05 | - | 64.70 ± 5.88 | - |
| **10 µM sanguinarine** | -1.57 ± 1.65 | - | -16.64 ± 2.55 | - |
| **1 µM sanguinarine** | 83.32 ± 6.83 | - | -2.80 ± 3.67 | - |
| **10 µM chelerythrine** | 0.87 ± 0.74 | - | 0.63 ± 8.46 | - |
| **1 µM chelerythrine** | 107.44 ± 10.46 | - | 63.92 ± 4.30 | - |
| **10 µM protopine** | 103.87 ± 19.52 | - | 73.10 ± 0.51 | - |
| **0.1% Pa_Gli_20°C_28d** | - | - | 154.48 ± 19.00 | |
| **0.1% Fi_CGli_20°C_21d** | 103.99 ± 4.93 | - | 191.49 ± 16.20 | - |

Table 5 shows the effect of *C. majus* extracts at 0.02%v/v concentration on mitochondrial dehydrogenase activity in HDF, HUVEC and HEK cells.

**Table 5**

| ID example | Mean % response in proliferation ± SD on HUVEC | Mean % response in proliferation ± SD on HDF | Mean % response in proliferation ± SD on HEK |
|---|---|---|---|
| PA_Gli_20°C_28d | 105 ± 17 | 127 ± 16 | 130 ± 18 |
| Fi_f_HCl_20°C_21d | 142 ± 10 | 122 ± 9 | 161 ± 1 |
| Fi_LHCL20°C_7d | 143 ± 16 | 122 ± 4 | 136 ± 10 |

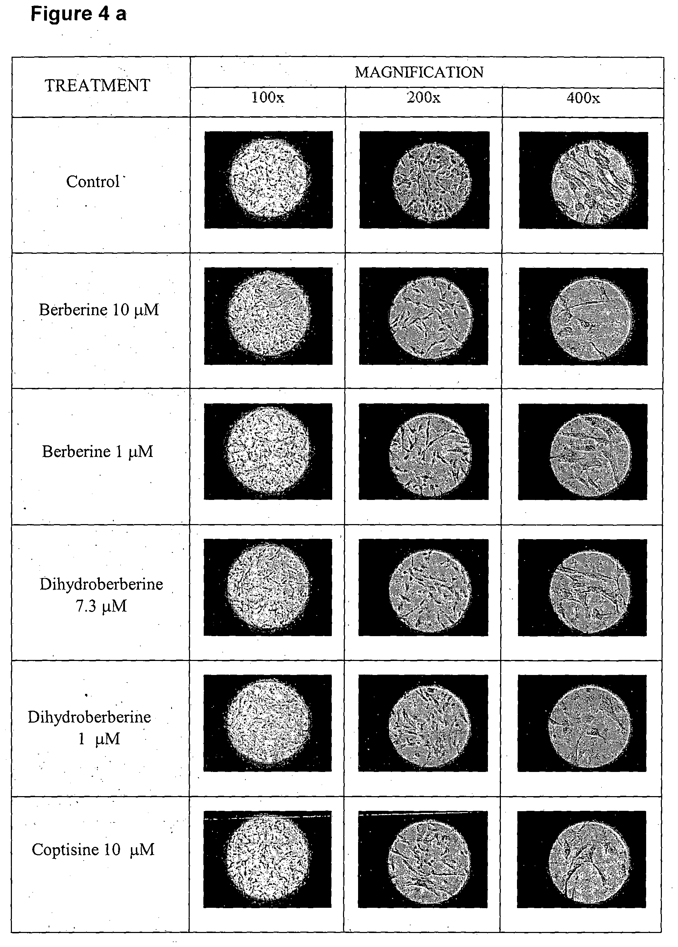

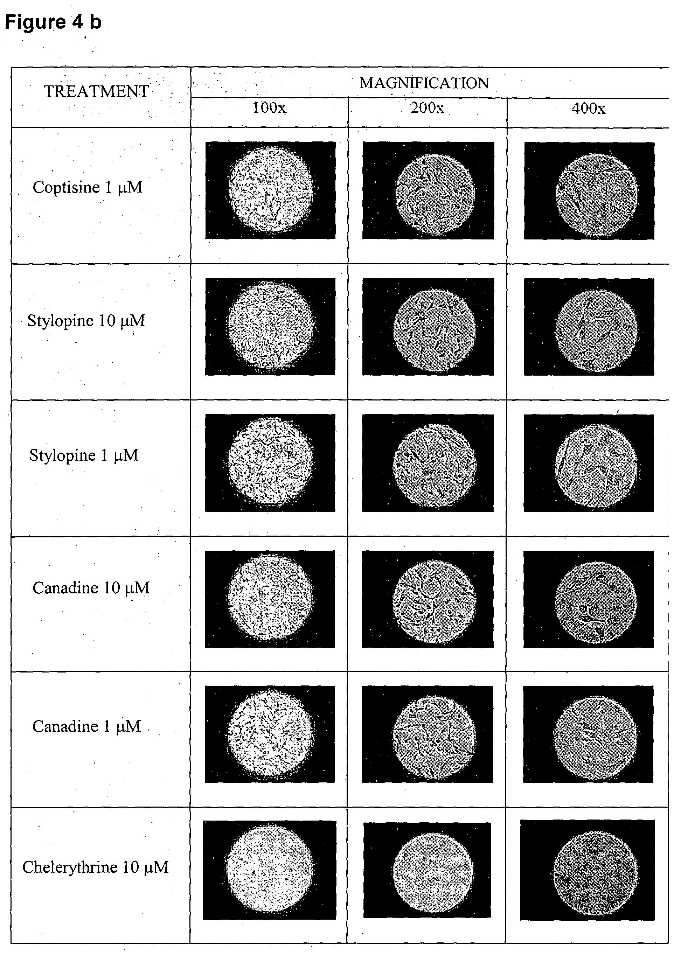

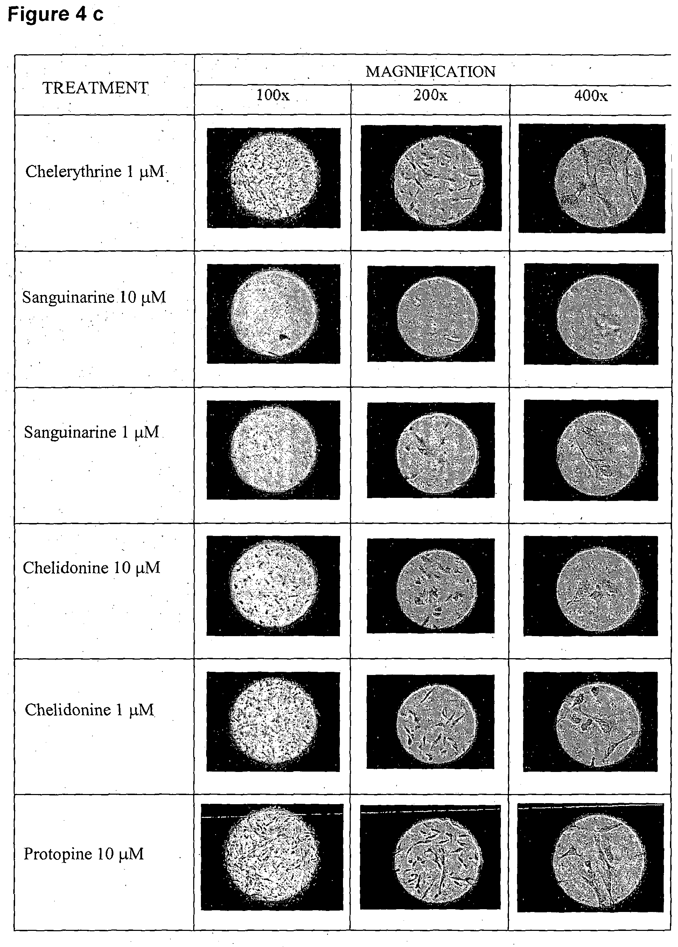

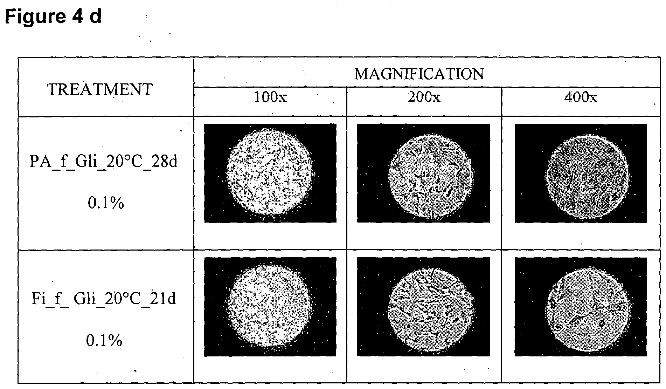

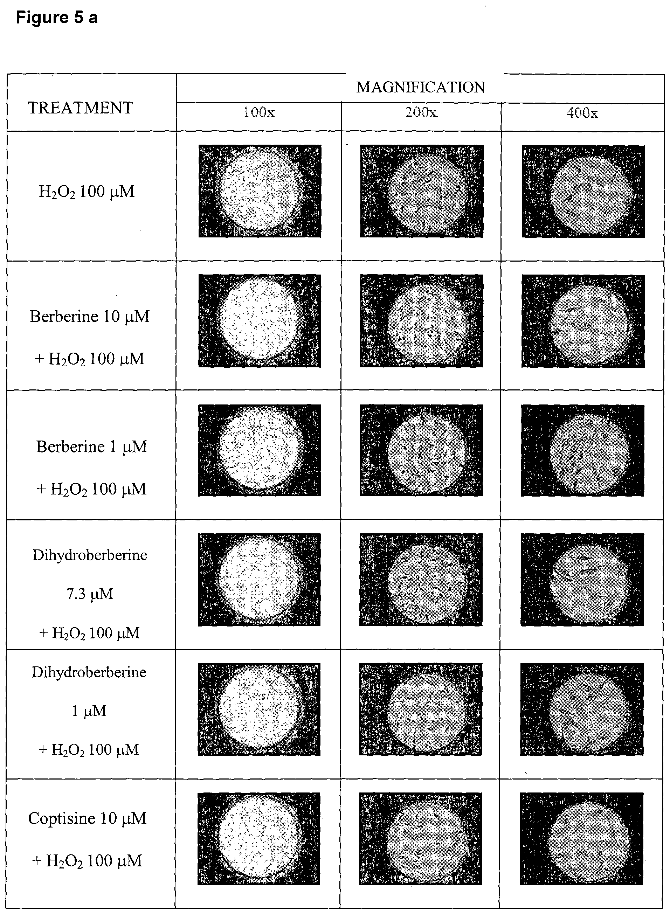

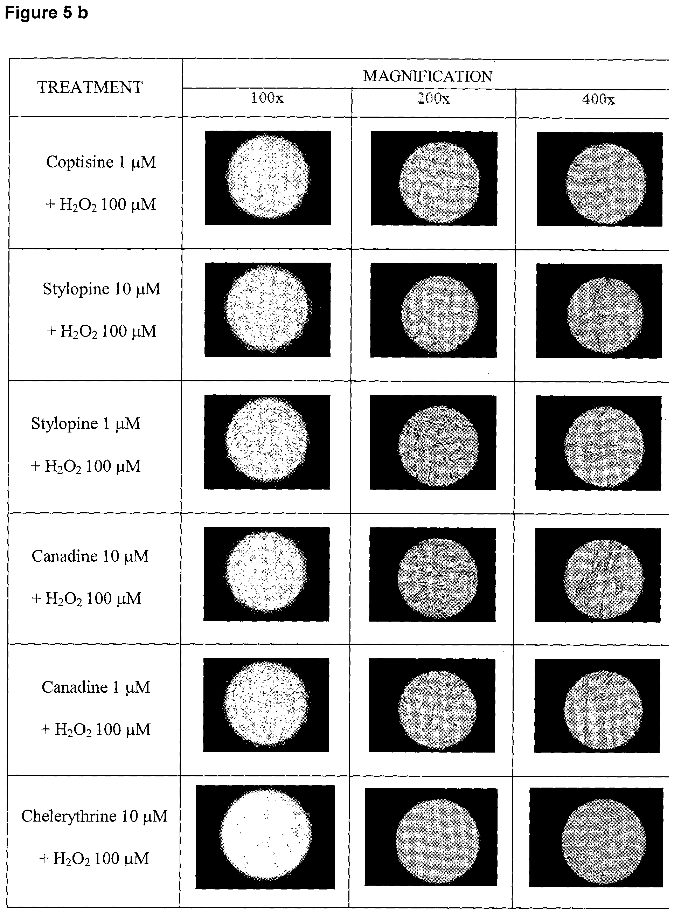

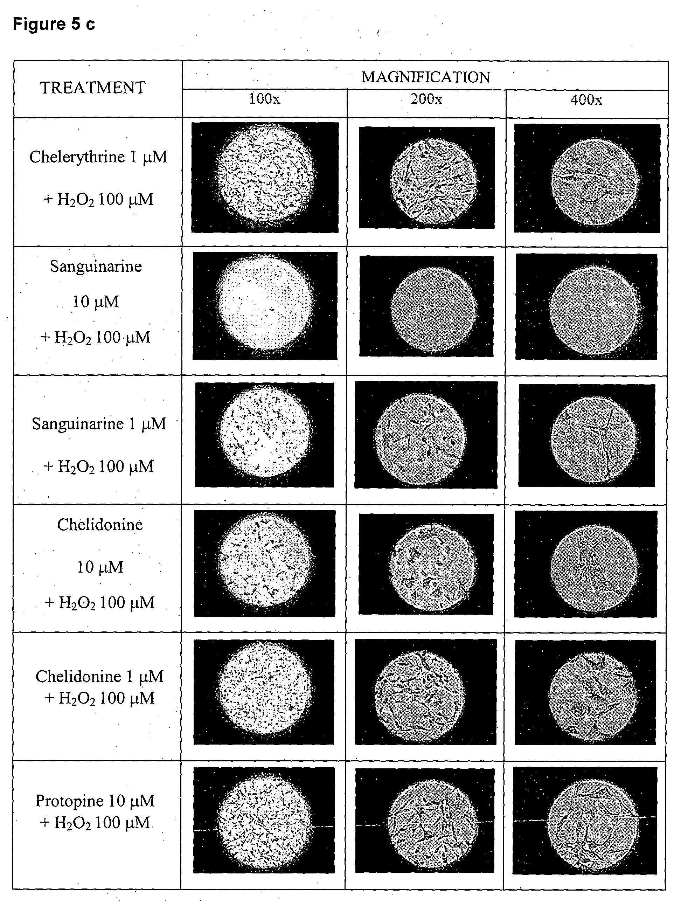

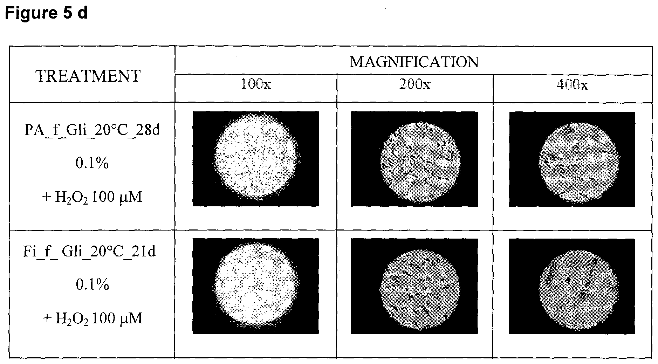

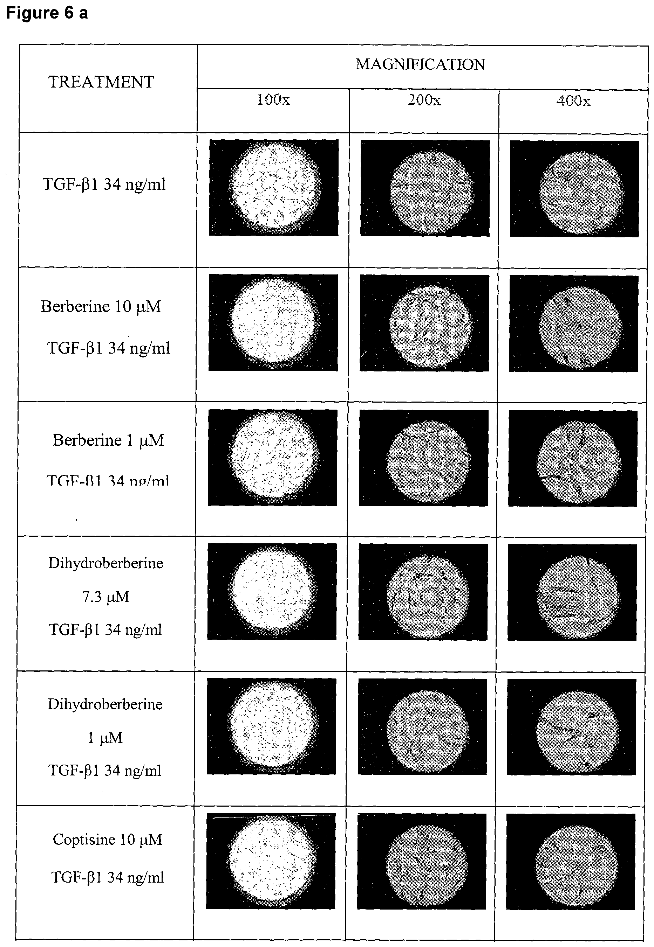

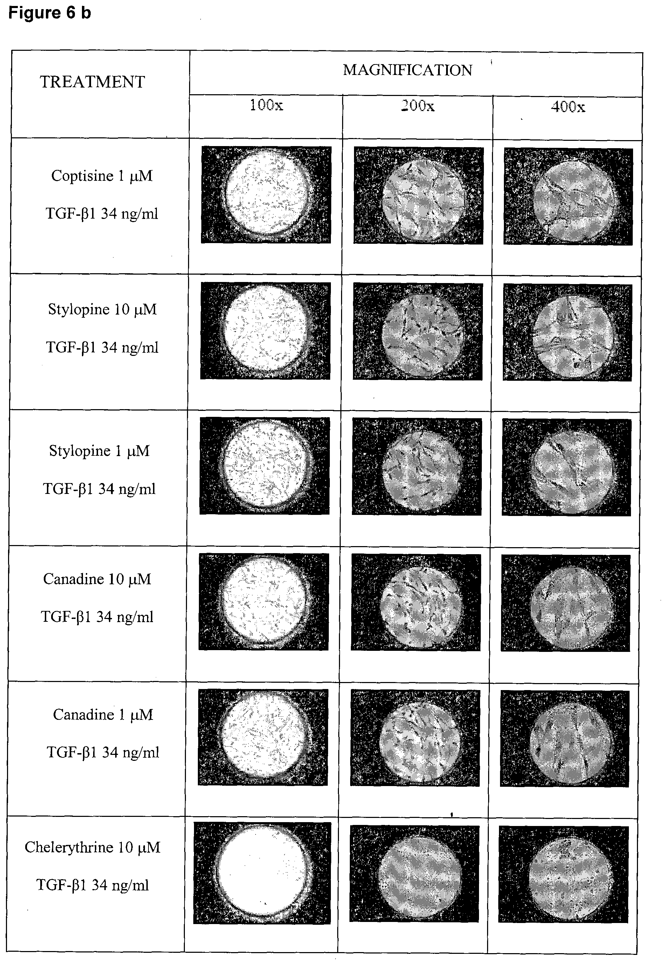

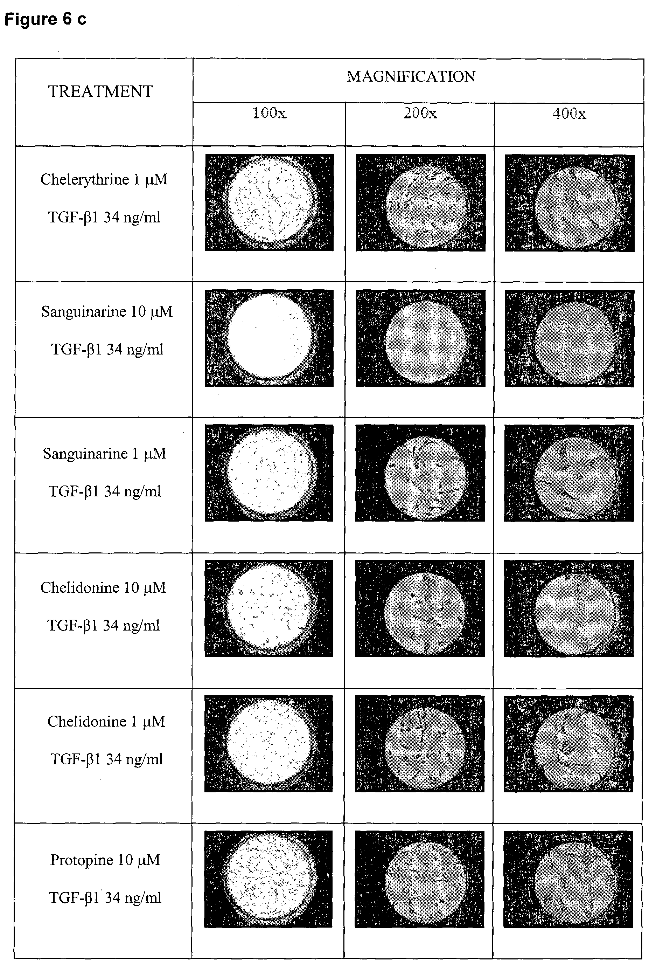

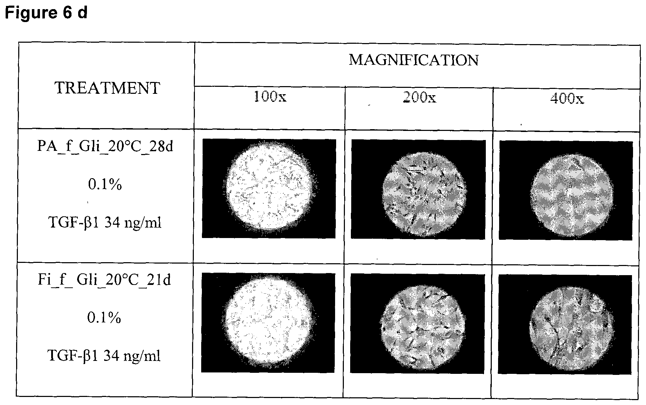

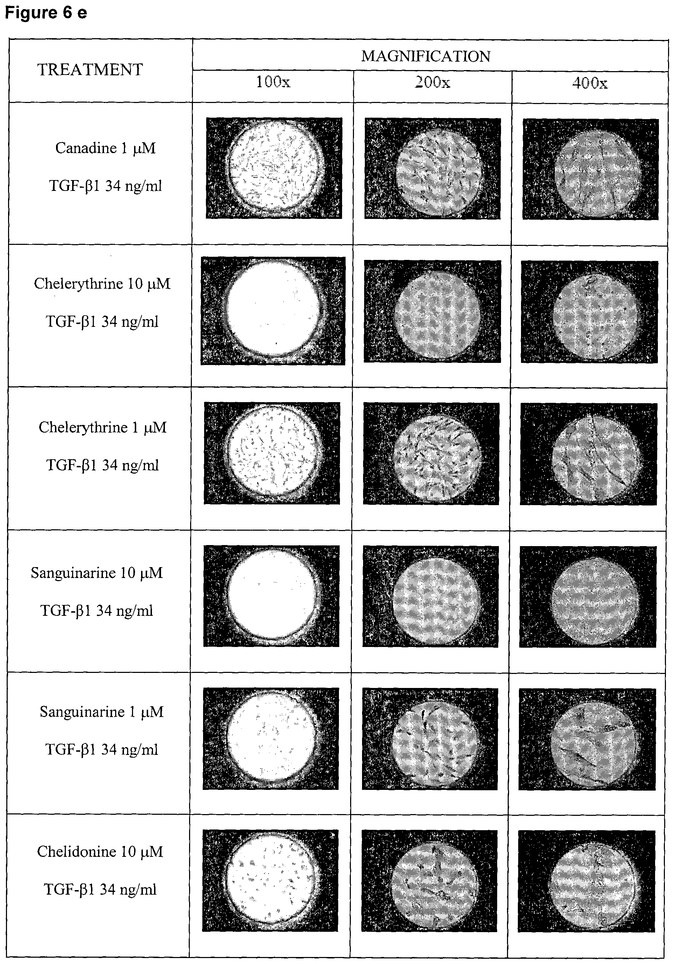

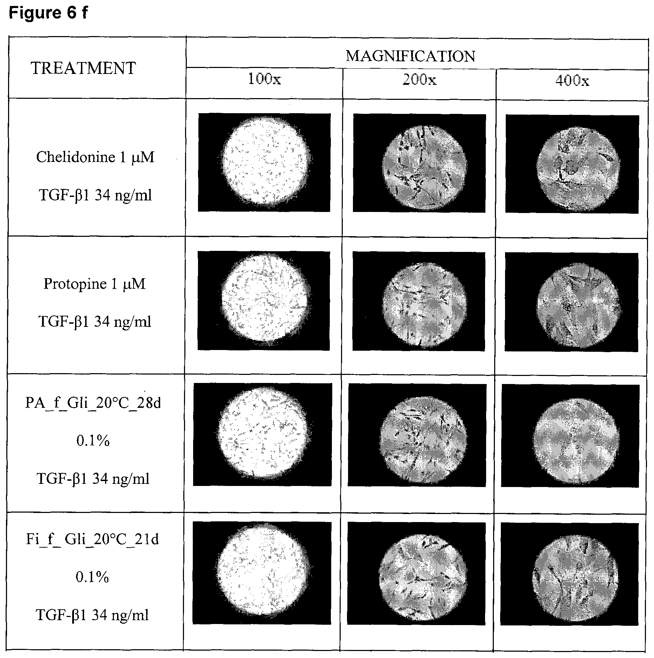

The results of the effects on cell morphology, produced by the treatment with the test compounds for 24 hours at the concentrations specified, in HDF cells stained for collagen with PSR are shown in Figures 4a-d, 5a-d and 6a-f. These Figures allow evaluating cell morphology through light microscopy observation at magnifications of 100x, 200x and 400x for HDF cells cultured under standard growth conditions, in the presence of 100 µM H₂O₂ and in the presence of 34 ng/ml TGF-β1.

The results of the biological effects concerning data about cell proliferation and data normalized for cell number about collagen, IL-1β and IL-6 production are reported in Tables 6 and 7.

**Table 6. Data concerning cell proliferation, collagen production and release of inflammatory cytokines obtained for HDF cells stained for collagen with Sirius Red and subjected to various treatments with the test compounds at the concentrations specified, for a period of 24 hours, under standard growth conditions, in the presence of 100 µM H₂O₂ and in the presence of 34 ng/ml TGF-β1. Cell proliferation and collagen production are expressed as the mean value from three experiments ± SD, while production of the inflammatory cytokines IL-1β and IL-6 is expressed as the mean value from two experiments ± SD.**

| **Treatment** | **Standard conditions** | | | | **100 µM H₂O₂** | | | | **34 ng/ml TGF-β1** | | | |
|---|---|---|---|---|---|---|---|---|---|---|---|---|
| | **No. of cells from photo^{a} ± SD** | **Abs^{∗}10^{- 6}/No. of cells from photo*^{b}* ± SD** | **IL-1β ng/(ml* No. of cells from photo)*^{c}* ± SD** | **IL-6** n**g/(ml* No. of cells from photo)*^{d}* ± SD** | **No. of cells from photo*^{a}* ± SD** | **Abs^{∗}10^{- 6}/No. of cells from photo*^{b}* ± SD** | **IL-1β ng/(ml* No. of cells from photo)^{c} ± SD** | **IL-6 ngl(ml* No. of cells from photo)*^{d}* ± SD** | **No. of cells from photo*^{a}* ± SD** | **Abs^{∗}10^{- 6}/No. of cells from photo*^{b}* ± SD** | **IL-1β ng/(ml* No. of cells from photo)*^{c}* ± SD** | **IL-6 ng/(ml* No. of cells from photo)*^{d}* ± SD** |
| Control (C) | 186±28 | 1720±102 | 112±1 | 715±32 | 183±17 | 1475±16 | 142±2 | 1295±109 | 163±4 | 2576±122 | 159±6 | 969±36 |
| 10 µM berberine | 176±35 | 1761±56 | 94±5 | 618±25 | 119±10 | 1764±75 | 210±16 | 1201±84 | 184±28 | 1739±141 | 146±4 | 603±21 |
| 1 µM berberine | 203±11 | 1527±24 | 120±6 | 843±35 | 129±24 | 1937±93 | 178±1 | 1519±155 | 181±18 | 1933±16 | 138±5 | 657±5 |
| 7.3 µM dihydroberberine | 129±13 | 2713±54 | 125±2 | 534±39 | 108±3 | 2129±27 | 212±18 | 1685±444 | 193±27 | 1865±150 | 129±1 | 704±60 |
| 1 µM dihydroberberine | 160±18 | 2312±118 | 108±1 | 541±24 | 146±26 | 1712±116 | 164±1 | 1328±2 | 232±13 | 1551±150 | 99±10 | 512±25 |
| 10 µM coptisine | 129±27 | 2558±38 | 155±7 | 751±93 | 114±0 | 2017±131 | 236±8 | 1710±114 | 86±6 | 3837±337 | 267±15 | 1244±58 |
| 1 µM coptisine | 144±12 | 2569±34 | 137±12 | 612±31 | 143±17 | 1748±34 | 174±20 | 1034±27 | 111±18 | 3243±234 | 207±8 | 1027±36 |
| 10 µM stylopine | 183±12 | 1967±98 | 186±7 | 961±38 | 249±8 | 963±72 | 88±10 | 449±2 | 210±17 | 1571±109 | 109±14 | 528±33 |
| 1 µM stylopine | 234± 7 | 1666±55 | 159±10 | 868±62 | 240±12 | 1166±41 | 100±4 | 583±25 | 237±19 | 1518±37 | 97±3 | 447±8 |
| 10 µM canadine | 138±5 | 2246±79 | 109±1 | 508±43 | 188±25 | 1329±85 | 132±138 | 718±63 | 138±3 | 2536±224 | 173±14 | 724±43 |
| 1 µM canadine | 207±12 | 1787±28 | 81±2 | 405±8 | 253±32 | 948±35 | 90±3 | 584±19 | 206±40 | 1699±53 | 116±19 | 451±121 |
| 10 µM chelerythrine | 3±3 | 24590±1639 | 144±4 | 572±14 | 10±5 | 9380±656 | 1735±469 | 5844±328 | 10±4 | 7786±375 | 1632±93 | 4652±243 |
| 1 µM chelerythrine | 229±20 | 1428±117 | 96±101 | 396±14 | 289±14 | 1556±24 | 69±20 | 626±8 | 201±15 | 1933±89 | 98±10 | 510±43 |
| 10 µM sanguinarine | 1±0 | 90000±0 | 4535±81 | 17786±27 | 1±0 | 72000±5000 | 18400±2800 | 66300±5000 | 1±0 | 84000±3000 | 16400±5000 | 56600±9000 |
| 1 µM sanguinarine | 30±9 | 4733±233 | 75±1 | 470±32 | 110±18 | 1509±117 | 253±9 | 2374±264 | 86±7 | 2651±46 | 297±13 | 2167±279 |
| 10 µM chelidonine | 109±16 | 2633±146 | 17500±700 | 38100±8300 | 101±4 | 1944±49 | 366±35 | 1990±32 | 79i7 | 3579±75 | 363±27 | 1231±76 |
| 1 µM chelidonine | 202±21 | 1742±34 | 666±30 | 2883±553 | 189±37 | 1294±36 | 115±8 | 697±87 | 184±25 | 2048±81 | 127±26 | 620±72 |
| 10 µM protopine | 306±18 | 1068±88 | 260±19 | 982±203 | 275±16 | 917±47 | 97±21 | 739±18 | 257±27 | 1463±23 | 107±6 | 435±98 |
| 0.1% Pa_f_G!i_20°C_28d | 176±29 | 1704±56 | 99±10 | 402±4 | 208±26 | 1802±33 | 99±10 | 924±7 | 163±18 | 2245±67 | 145±10 | 749±7 |
| 0.1% Fi_f_Gli_20°C_21d | 177±11 | 1694±96 | 69±2 | 281±65 | 206±14 | 2063±160 | 102±0 | 927±100 | 170±5 | 2158±70 | 159±15 | 797±48 |

| | | | | | | | | | | | | |
|---|---|---|---|---|---|---|---|---|---|---|---|---|
| *^{a}* No. of cells from photo = number of cells derived by microphotography, a parameter indicating cell proliferation *^{b}* Abs^{∗}10⁻⁶/No. of cells from photo = Absorbance scaled by a factor of 10⁻⁶ and related to the number of cells derived by microphotography, a parameter indicating collagen production per cell *^{c}* IL-1β ng/(ml^{∗}No. of cells from photo) = concentration of IL-1β expressed in ng/ml and related to the number of cells derived by microphotography, a parameter indicating IL-1β production per cell *^{d}* IL-6 ng/(ml^{∗}No. of cells from photo) = concentration of IL-6 expressed in ng/ml and related to the number of cells derived by microphotography, a parameter indicating IL-6 production per cell SD = standard deviation | | | | | | | | | | | | |

**Table 7. Comparison between the means for cell proliferation, collagen production and release of inflammatory cytokines reported in Table 6, expressed as probability (P) obtained by the Student's t-test. Probability values ≤0.05 are reported in bold.**

| **Treatment** | **Standard conditions - Value of P vs. Control** | | | | **100 µM H₂O₂ - Value of P vs. Control + H₂O₂** | | | | **34 ng/ml TGF-β1** - **Value of P vs. Control + TGF-β1** | | | |
|---|---|---|---|---|---|---|---|---|---|---|---|---|
| | **No. of cells from photo^{a}** | **Abs^{∗}10⁻⁶/No. of cells from photo*^{b}*** | **IL-1β ng/(ml^{∗} No. of cells from photo)*^{c}*** | **IL-6 ng/(ml^{∗} No. of cells from photo)*^{d}*** | **No. of cells from photo^{a}** | **Abs^{∗}10⁻⁶/No. of cells from photo*^{b}*** | **IL-1β ng/(ml^{∗} No. of cells from photo)*^{c}*** | **IL-6 ng/(ml^{∗} No. of cells from photo)*^{d}*** | **No. of cells from photo*^{a}*** | **Abs^{∗}10 ⁶/No. of cells from photo*^{b}*** | **IL-1β ng/(ml^{∗} No. of cells from photo)*^{c}*** | **IL-6 ng/(ml^{∗} No. of cells from photo)*^{d}*** |
| Control (C) | - | - | - | - | - | - | - | - | - | - | - | - |
| Control + H₂O₂ | 0.8816 | **0.0147** | **0.0028** | **0.0186** | - | - | - | - | - | - | - | - |
| Control + TGF-β1 | 0.2318 | **0.0007** | **0.0083** | **0.0175** | - | - | - | - | - | - | - | - |
| 10 µM berberine | 0.7189 | 0.5746 | **0.0145** | **0.0367** | **0.0049** | **0.0028** | **0.027** | 0.436 | 0.2678 | **0.0015** | 0.1255 | **0.0064** |
| 1 µM berberine | 0.3831 | **0.0332** | 0.0572 | **0.0254** | **0.0335** | **0.0011** | **0.0019** | 0.2365 | 0.1661 | **0.0008** | 0.0627 | **0.0067** |
| 7.3 µM dihydroberberine | **0.033** | **0.0001** | **0.0133** | 0.6563 | **0.0017** | **P<0.0001** | **0.0319** | 0.351 | 0.1297 | **0.0031** | **0.0199** | **0.0331** |
| 1 µM dihydroberberine | 0.2475 | **0.0028** | 0.0991 | 0.0822 | 0.1081 | **0.0248** | **0.0051** | 0.7103 | **0.0009** | **0.0008** | **0.0184** | **0.0046** |
| 10 µM coptisine | 0.0641 | **0.0002** | **0.0045** | **0.0198** | **0.0022** | **0.0021** | **0.0038** | 0.0652 | **0.0001** | **0.0037** | **0.011** | **0.0295** |
| 1 µM coptisine | 0.0753 | **0.0002** | **0.0221** | 0.0901 | **0.0449** | **0.0002** | 0.1532 | 0.0814 | **0.0081** | **0.0119** | **0.021** | 0.2485 |
| 10 µM stylopine | 0.8728 | **0.039** | 0.0955 | **0.0319** | **0.0037** | **0.0003** | **0.0009** | **0.0082** | **0.0096** | **0.0004** | **0.0434** | **0.0061** |
| 1 µM stylopine | **0.045** | 0.4649 | **0.0026** | **0.0056** | **0.009** | **0.0003** | **0.0056** | **0.0121** | **0.0027** | **0.0001** | **0.0058** | **0.0025** |
| 10 µM canadine | **0.0431** | **0.0021** | **0.0082** | **0.0286** | 0.7888 | **0.0431** | 0.9277 | **0.023** | **0.001** | 0.7994 | 0.3233 | **0.0252** |
| 1 µM canadine | 0.2984 | 0.3342 | 0.8435 | **0.0059** | **0.0287** | **P<0.0001** | **0.0024** | **0.0119** | 0.1376 | **0.0003** | 0.0927 | **0.0284** |
| 10 µM chelerythrine | **0.0004** | **P<0.0001** | **0.0002** | **P<0.0001** | **0.0001** | **P<0.0001** | **0.0407** | **0.0029** | **P<0.0001** | **P<0.0001** | **0.002** | **0.0022** |
| 1 µM chelerythrine | 0.096 | **0.0311** | **0.0007** | **0.0166** | **0.0012** | **0.0083** | **0.0359** | **0.0131** | 0.0141 | **0.0018** | **0.0178** | **0.0074** |
| 10 µM sanguinarine | **P<0.0001** | **P<0.0001** | **0.0008** | **0.0238** | **0.0001** | **P<0.0001** | **0.0116** | **0.0029** | **P<0.0001** | **P<0.0001** | **0.0443** | **0.0128** |
| 1 µM sanguinarine | **0.0008** | **P<0.0001** | **0.0015** | **0.0311** | **0.0075** | 0.6442 | **0.0034** | **0.0333** | **P<0.0001** | 0.3755 | **0.0053** | **0.0265** |
| 10 µM chelidonine | **0.0147** | **0.0009** | **0.0082** | 0.2076 | **0.0013** | **P<0.0001** | **0.012** | **0.0131** | **P<0.0001** | **0.0003** | **0.0091** | **0.0478** |
| 1 µM chelidonine | 0.4747 | 0.7409 | **0.0059** | **0.0053** | 0.8033 | **0.0014** | **0.0436** | **0.0261** | 0.2302 | **0.0034** | 0.232 | **0.0256** |
| 10 µM protopine | **0.0034** | **0.0011** | **0.0013** | **0.0136** | **0.0025** | **P<0.0001** | 0.0946 | **0.0192** | **0.004** | **0.0001** | **0.0131** | **0.0186** |
| 0.1% Pa_f_GL20°C_28d | 0.6896 | 0.8235 | 0.2947 | 0.0918 | 0.2358 | **P<0.0001** | **0.027** | **0.0407** | 1 | **0.0146** | 0.2317 | **0.0136** |
| 0.1% Fi_f_Gli_20°C_21d | 0.6317 | 0.7639 | 0.4226 | 0.0636 | 0.1447 | **0.0032** | **0.0016** | 0.0722 | 0.1312 | **0.0068** | 1 | 0.0558 |

| | | | | | | | | | | | | |
|---|---|---|---|---|---|---|---|---|---|---|---|---|
| *^{a}* No. of cells from photo = number of cells derived by microphotography, a parameter indicating cell proliferation ^{b} Abs^{∗}10⁻⁶/No. of cells from photo = Absorbance scaled by a factor of 10⁻⁶ and related to the number of cells derived by microphotography, a parameter indicating collagen production per cell *^{c}* IL-1β ng/(ml^{∗}No. of cells from photo) = concentration of IL-1β expressed in ng/ml and related to the number of cells derived by microphotography, a parameter indicating IL-1β production per cell *^{d}* IL-6 ng/(ml^{∗}No. of cells from photo) = concentration of IL-6 expressed in ng/ml and related to the number of cells derived by microphotography, a parameter indicating IL-6 production per cell SD = standard deviation | | | | | | | | | | | | |

Table 8 shows the effect of the most active *Chelidonium majus* extracts on the migration of HDF and HUVEC cells.

**Table 8**

| **ID example** | **Percentage cell area of HUVEC ± SD at 24 hours** | **Percentage cell area of HUVEC ± SD at 48 hours** | **Percentage cell area of HDF ± SD at 24 hours** | **Percentage cell area of HDF ± SD at 48 hours** |
|---|---|---|---|---|
| **Control** | 22 ± 7 | 30 ± 5 | 32 ± 1 | 48 ± 4 |
| Fi_HCl_20°C_7d | 37 ± 4 | 39 ± 4 | 38 ± 3 | 64 ± 0 |

### Anti-radical activity of C. majus alkaloids and extracts

Tables 9 and 10 report the anti-radical activity values of the individual *C. majus* alkaloids and extracts. In particular, the compounds of interest were initially tested at a single concentration in order to identify which of them had the most marked anti-radical properties. The results of this first screening are reported in Table 9.

**Table 9. Anti-radical activity - first screening at a single concentration. The data refers to the mean of three independent experiments.**

| **Substance** | **% DPPH activity** | **SEM** |
|---|---|---|
| **Controls** | | |
| Negative control, Ethanol | 100 | 1 |
| Positive control, 0.1 mM vitamin C | 0 | 1 |

| ***C. majus alkaloids*** | | |
|---|---|---|
| 1mM canadine | 103 | 10 |
| 1 mM berberine | 100 | 9 |
| 1 mM coptisine | 114 | 7 |
| 10mM sanguinarine | 172 | 9 |
| 1 mM dihydroberberine | 55 | 16 |
| 1 mM chelerythrine | 115 | 10 |
| 1 mM stylopine | 113 | 11 |
| 1 mM protopine | 99 | 5 |
| 1 mM chelidonine | 98 | 8 |

| ***C. majus extracts*** | | |
|---|---|---|
| Fo-ner_f_Aq_45°C_24h 1% | 81 | 7 |
| Fi_f_Aq_45°C_24h 1% | 100 | 17 |
| Fi_s_Aq_45°C_24h 1% | 131 | 33 |
| Fi_f_HCl_20°C_21d 1% | 62 | 11 |
| Fi_f_Gli_20°C_21d 1% | 20 | 5 |
| PA_f_Gli_20°C_28d 1% | 78 | 6 |
| 1% propylene glycol | 100 | 1 |

Based on the analysis of the results reported in Table 9, some substances were selected for further characterisation of their anti-radical activity. For the selected substances, dose-activity curves were obtained and the IC₅₀ value were computed using a non-linear regression model with the Hill equation at variable slope. Such substances had been selected due to both their anti-radical potential and the easiness of their isolation/production. The selection of substances was made in view of cosmetics applications. Moreover their activity on human dermal fibroblasts (HDF) is expected to be useful also for the development of dermatological medications. The selected substances are discussed below.

### C. majus alkaloids:

Dihydroberberine

### C. maius extracts:

Fi_f_Gli_20°C_21d (glycol extract of fresh flowers, with high antioxidant and anti-radical activity. Among the glycol extracts is the one possessing the greatest stimulation of cell proliferation and optimal preservability at room temperature for long periods of time);
Fi_f_HCl_20°C_21d (extract with high antioxidant and anti-radical activity. Among the aqueous extracts is the one possessing the greatest stimulation of cell proliferation together with optimal preservability at room temperature for long periods of time);

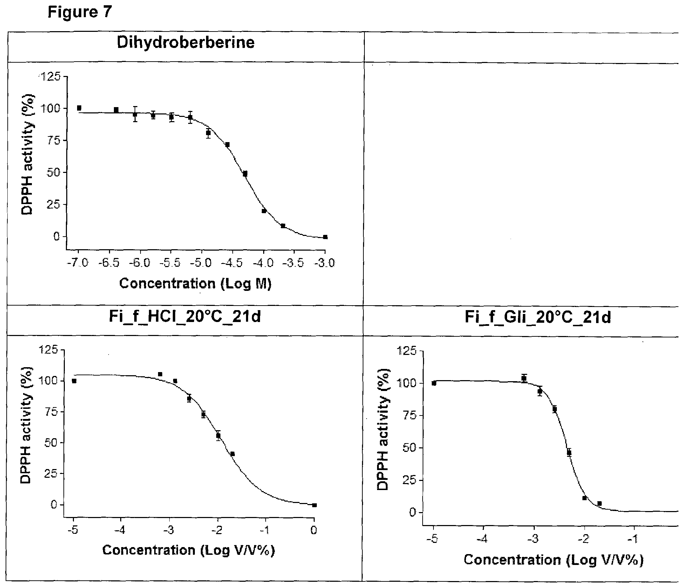

For each of the substances selected for further investigation of anti-radical activity, a representative dose-anti-radical activity curve is reported. (See Figure 7).

Table 10 reports the IC₅₀ values of the substances selected for further investigation of anti-radical activity.

**Table 10. IC₅₀ values of the substances selected for deep investigation of anti-radical activity. The values represent the mean of at least two independent experiments.**

| **Compound** | **IC50 µM** |
|---|---|
| Dihydroberberine | 62±13 |
| | |

| **Extract** | **IC50 %v/v** |
|---|---|
| Fi_f_Gli_20°C_21d | 0.51 ± 0.06 |
| Fi_f_HCl_20°C_21d | 1.02 ± 0.16 |

From the detailed description of the Examples reported above, the advantages obtained by the *Chelidonium majus* extracts and the present invention are evident. In particular, said extracts showed to be surprisingly and advantageously well suited to use in the cosmetic and pharmaceutical fields, and in particular for use in the treatment of skin regeneration or skin dysfunction repair.

## Claims

1. Process for the preparation of a *Chelidonium majus* extract, said process comprising the following steps:
a. bringing the aerial parts selected from the group consisting of flowers, flower-buds, buds and leaves without nervation of the *Chelidonium majus* plant into contact with an extraction solvent, selected from the group consisting of propylene glycol, and aqueous solutions of HCl, at a temperature from 20°C to 50 °C;
b. recovering the extract from step a,
wherein the Chelidonium majus extract comprising protopine, stylopine, chelidonine, sanguinarine, chelerythrine, berberine, coptisine, wherein:
the ratio between the quantity expressed in weight of stylopine with respect to the sum of the quantities expressed in weight of chelidonine, sanguinarine and chelerythrine is greater than or equal to 5;
the ratio between the quantity expressed in weight of berberine with respect to the sum of the quantities expressed in weight of chelidonine, sanguinarine and chelerythrine is greater than or equal to 1; and;
the ratio between the quantity expressed in weight of berberine with respect to the quantity expressed in weight of coptisine is greater than or equal to 0.04.

2. The process according to claim 1, wherein said extract is obtained by maceration at 20°C of said aerial parts of step a. in propylene glycol.

3. The process according to claim 1, wherein said extract is obtained by maceration at 20°C of said flowers of step a. in 5% HCl.

4. The process according to claim 1, wherein said extract is obtained by maceration at 20°C of said flowers of step a. in propylene glycol.

5. A *Chelidonium majus* extract obtainable from the process of anyone of claims 1 to 4.

6. The *Chelidonium majus* extract according to claim 5, for use as a medicament in the treatment of skin regeneration or repair disorders.

7. A cosmetic composition comprising the *Chelidonium majus* the extract according to claim 5 and a cosmetically acceptable excipient.

8. A pharmaceutical composition comprising the *Chelidonium majus* extract according to claim 5 and a pharmaceutically acceptable excipient.

## Patentansprüche

1. Verfahren zur Herstellung eines Extrakts von *Chelidonium majus,* wobei das Verfahren folgende Schritte umfasst:
a. Inkontaktbringen der aus der Gruppe bestehend aus Blüten, Blütenknospen, Knospen und Blättern ohne Aderung der *Chelidonium majus*-Pflanze ausgewählten Luftteile mit einem Extraktionslösungsmittel, ausgewählt aus der Gruppe bestehend aus Propylenglykol und wässrigen Lösungen von HCl, bei einer Temperatur von 20 °C bis 50 °C,
b. Gewinnen des Extrakts aus Schritt a,
wobei der Extrakt von Chelidonium majus Protopin, Stylopin, Chelidonin, Sanguinarin, Chelerythrin, Berberin, Coptisin umfasst, wobei:
das Verhältnis zwischen der Menge, ausgedrückt in Gewicht von Stylopin, bezogen auf die Summe der in Gewicht von Chelidonin, Sanguinarin und Chelerythrin ausgedrückten Mengen größer als oder gleich 5 ist,
das Verhältnis zwischen der Menge, ausgedrückt in Gewicht von Berberin, bezogen auf die Summe der in Gewicht von Chelidonin, Sanguinarin und Chelerythrin ausgedrückten Menge größer als oder gleich 1 ist; und;
das Verhältnis zwischen der in Gewicht von Berberin ausgedrückten Menge, bezogen auf die in Gewicht von Coptisin ausgedrückten Menge größer als oder gleich 0,04 ist.

2. Verfahren nach Anspruch 1, wobei der Extrakt durch Mazeration der Luftteile von Schritt a. in Propylenglykol bei 20 °C gewonnen wird.

3. Verfahren nach Anspruch 1, wobei der Extrakt durch Mazeration der Blumen von Schritt a. in 5 % HCl bei 20 °C gewonnen wird.

4. Verfahren nach Anspruch 1, wobei der Extrakt durch Mazeration der Blumen von Schritt a. in Propylenglykol bei 20 °C gewonnen wird.

5. Extrakt von *Chelidonium majus,* erhältlich aus dem Prozess nach einem der Ansprüche 1 bis 4.

6. Extrakt von *Chelidonium majus* nach Anspruch 5 zur Verwendung als Arzneimittel bei der Behandlung von Hautregenerations- oder Reparaturstörungen.

7. Kosmetische Zusammensetzung, umfassend den Extrakt von *Chelidonium majus* nach Anspruch 5 und einen kosmetisch verträglichen Hilfsstoff.

8. Pharmazeutische Zusammensetzung, umfassend den Extrakt von *Chelidonium majus* nach Anspruch 5 und einen pharmazeutisch verträglichen Hilfsstoff.

## Revendications

1. Procédé de préparation d'un extrait de *Chelidonium majus,* ledit procédé comprenant les étapes suivantes :
a. mise en contact des parties aériennes choisies dans le groupe constitué des fleurs, des bourgeons floraux, des bourgeons et des feuilles sans nervation de la plante *Chelidonium majus* avec un solvant d'extraction choisi dans le groupe constitué du propylène glycol et des solutions aqueuses d'HCl, à température de 20 °C à 50 °C ;
b. récupération de l'extrait de l'étape a,
dans lequel l'extrait de *Chelidonium majus* comprend la protopine, la stylopine, la chélidonine, la sanguinarine, la chellérithrine, la berbérine, la coptisine, dans lequel :
le rapport entre la quantité exprimée en poids de stylopine par rapport à la somme des quantités exprimées en poids de chélidonine, de sanguinarine et de chellérythrine est supérieur ou égal à 5 ;
le rapport entre la quantité exprimée en poids de berbérine par rapport à la somme des quantités exprimées en poids de chélidonine, de sanguinarine et de chellérythrine est supérieur ou égal à 1 ; et ;
le rapport entre la quantité exprimée en poids de berbérine par rapport à la quantité exprimée en poids de coptisine est supérieur ou égal à 0,04.

2. Procédé selon la revendication 1, dans lequel ledit extrait est obtenu par macération à 20 °C desdites parties aériennes de l'étape a. dans le propylène glycol.

3. Procédé selon la revendication 1, dans lequel ledit extrait est obtenu par macération à 20 °C desdites fleurs de l'étape a. dans du HCl à 5 %.

4. Procédé selon la revendication 1, dans lequel ledit extrait est obtenu par macération à 20 °C desdites fleurs de l'étape a. dans le propylène glycol.

5. Extrait de *Chelidonium majus* pouvant être obtenu à partir du procédé de l'une quelconque des revendications 1 à 4.

6. Extrait de *Chelidonium majus* selon la revendication 5, pour une utilisation en tant que médicament dans le traitement de troubles de la régénération ou de la réparation de la peau.

7. Composition cosmétique comprenant l'extrait de *Chelidonium majus* selon la revendication 5 et un excipient cosmétiquement acceptable.

8. Composition pharmaceutique comprenant l'extrait de *Chelidonium majus* selon la revendication 5 et un excipient pharmaceutiquement acceptable.
